(19) Europäisches Patentamt
European Patent Office
Office européen des brevets

(11) Publication number: 0 270 494 B1

(12) EUROPEAN PATENT SPECIFICATION

(45) Date of publication of patent specification: **19.08.92**

(51) Int. Cl.[5]: **C07D 471/04**, C07D 471/10, A61K 31/435, //(C07D471/04, 231:00,221:00)

(21) Application number: **87810678.0**

(22) Date of filing: **19.11.87**

The file contains technical information submitted after the application was filed and not included in this specification

(54) **Certain cycloalka- [b]-pyrazolo-[3,4-d]-pyridin-3-one derivatives.**

(30) Priority: **25.11.86 US 934754**

(43) Date of publication of application: **08.06.88 Bulletin 88/23**

(45) Publication of the grant of the patent: **19.08.92 Bulletin 92/34**

(84) Designated Contracting States: **AT BE CH DE ES FR GB GR IT LI LU NL SE**

(56) References cited: **EP-A- 0 168 350**

(73) Proprietor: **CIBA-GEIGY AG**
**Klybeckstrasse 141**
**CH-4002 Basel(CH)**

(72) Inventor: **Yokoyama, Naokata**
**200 Winston Drive (Apt. 2103)**
**Cliffside New Jersey 07010(US)**

EP 0 270 494 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

EP-A-168 350 describes 2-substituted [b]-ring fused pyrazolo[3,4-d]pyridin-3-ones useful as benzodiazepine receptor modulators, e.g. with anxiolytic activity and EP-A-115 469 relates to unsaturated heterocyclic[b]fused pyrazolo[3,4-d]pyridin-3-ones particularly 2-aryl-pyrazolo[4,3-c], [1,6], [1,7] or [1,8]-naphthydrin-3-ones with anxiolytic and/or anticonvulsant activities.

This invention is directed to new 2-substituted 8 to 12 membered cycloalka-[b]-ring-fused pyrazolo[3,4-d]-pyridin-3-ones which are benzodiazepine receptor ligands and modulators, e.g. with anxiolytic activities and surprisingly unexpected less side-effects, processes for preparing the same and pharmaceutical composition comprising said compounds.

Particularly the invention relates to compounds of formula IA or IB

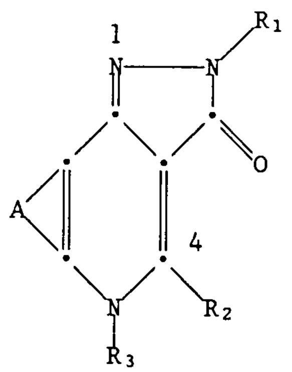

(IA)

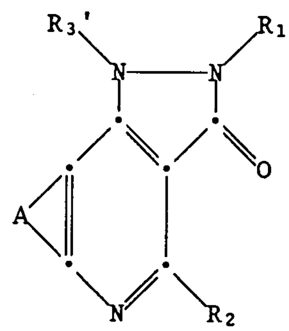

(IB)

wherein A represents an optionally substituted saturated divalent grouping which together with the two carbon atoms to which it is attached represents a fused 8, 9, 10, 11 or 12 membered carbocyclic ring selected from cycloocteno, cyclononeno, cyclodeceno, cycloundeceno and cyclododeceno; each unsubstituted or mono-, di-, tri-or tetra-substituted on carbon atoms within A by lower alkyl, lower alkylidene, $C_3$-$C_7$-cycloalkyl, hydroxy, lower alkanoyloxy, benzoyloxy unsubstituted or substituted by one or two lower alkoxy alkyl, lower alkoxy, halogen or trifluoromethyl, oxo, lower alkoxy, aryl or aryl-lower alkoxy; and when disubstituted on the same carbon atom within A, said carbon atom in each ring is preferably substituted by two lower alkyl or two aryl-lower alkyl groups, or by one lower alkyl or aryl-lower alkyl group and one group selected from hydroxy, lower alkoxy, aryl-lower alkoxy the term aryl within the above definitions represents phenyl or phenyl mono- or disubstituted by lower alkyl, lower alkoxy, hydroxy, lower alkanoyloxy, benzoyloxy unsubstituted or substituted by one or two lower alkyl, lower alkoxy, halogen or trifluoromethyl and lower alkanoyloxy, benzoyloxy unsubstituted or substituted by one or two lower alkoxy alkyl, lower alkoxy, halogen or trifluormethyl or each disubstituted on the same carbon atom within A by straight chain alkylene of 2 to 6 carbon atoms forming with the carbon to which the alkylene chain is attached a spiro-fused 3 to 7 membered ring; or each ring is disubstituted on adjacent carbon atoms by alkylene of 3,4 or 5 carbon atoms to form with the two adjacent carbon atoms to which said alkylene grouping is attached a fused 5-, 6-, or 7-membered ring; $R_1$ represents lower alkyl, phenyl, or phenyl mono- or disubstituted by lower alkyl, lower alkoxy, halogen or trifluoromethyl; or $R_1$ represents an aromatic heterocyclic radical selected from pyridyl, quinolyl, isoquinolyl, pyrimidyl and thiazolyl, or any said heterocyclic radical mono- or di-substituted on carbon by lower alkyl, lower alkoxy or halogen; and $R_2$, $R_3$ and $R_3'$ independently represent hydrogen or lower alkyl; or a pharmaceutically acceptable salt thereof, the term "lower" defines such with up to and including 7 carbon atoms.

Further preferred are said compounds of formula IA or IB wherein A together with the two carbon atoms to which it is attached represents a fused ring selected from cycloocteno, cyclononeno, cyclodeceno, cycloundeceno and cyclododeceno in which A represents hexamethylene, heptamethylene, octamethylene, nonamethylene or decamethylene respectively; each said ring unsubstituted or mono-, di-, tri- or tetra-substituted on carbon atoms within A by lower alkyl, lower alkylidene, $C_3$-$C_7$-cycloalkyl, hydroxy, lower alkanoyloxy, benzoyloxy unsubstituted or substituted by one or two lower alkoxy alkyl, lower alkoxy, halogen or trifluormethyl, oxo, lower alkoxy, aryl, aryl-lower alkyl or aryl-lower alkoxy; and when disubstituted on the same carbon atom within A, said carbon atom in each ring is preferably substituted by two lower alkyl or two aryl-lower alkyl groups, or by one lower alkyl or aryl-lower alkyl and one group selected from hydroxy, lower alkoxy, aryl-lower alkoxy the term aryl within the above definitions represents phenyl or

phenyl mono- or disubstituted by lower alkyl, lower alkoxy, hydroxy, lower alkanoyloxy, benzoyloxy unsubstituted or substituted by one or two lower alkyl, lower alkoxy, halogen or trifluoromethyl and lower alkanoyloxy, benzoyloxy unsubstituted or substituted by one or two lower alkoxy alkyl, lower alkoxy, halogen or trifluormethyl; or each ring is disubstituted on the same carbon atom within A by ethylene, propylene, butylene or pentylene forming with the carbon to which the alkylene chain is attached a spiro fused cyclopropyl, cyclobutyl, cyclopentyl or cyclohexyl ring; or each ring is disubstituted on adjacent carbon atoms by propylene or butylene to form with the two adjacent carbon atoms to which said alkylene grouping is attached a fused cyclopentane or cyclohexane ring; $R_1$, $R_2$, $R_3$ and $R_3'$ have meaning as defined in claim 2; or a pharmaceutically acceptable salt thereof, the term "lower" defines and with up to and including 7 carbon atoms.

A further preferred embodiment of the invention is represented by the $R_1$-substituted $C_8$-$C_{12}$-cycloalka-[b]-pyrazolo[3,4-d]pyridin-3-ones of the formula II

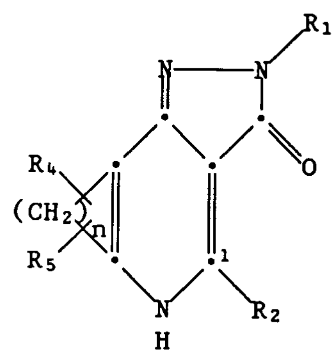

(II)

wherein $R_1$ represents lower alkyl, phenyl or phenyl mono- or di-substituted by lower alkyl, lower alkoxy, halogen or trifluoromethyl; or $R_1$ represents an aromatic heterocyclic radical selected from pyridyl, quinolyl, isoquinolyl, pyrimidyl and thiazolyl, or any said radical mono- or di-substituted by lower alkyl, lower alkoxy or halogen; $R_4$ and $R_5$ represent independently hydrogen, lower alkyl, $C_3$-$C_7$-cycloalkyl, hydroxy, lower alkanoyloxy, benzoyloxy unsubstituted or substituted by one or two lower alkoxy alkyl, lower alkoxy, halogen or trifluormethyl, lower alkoxy, aryl, aryl-lower alkyl or aryl-lower alkoxy the term aryl within the above definitions represents phenyl or phenyl mono- or disubstituted by lower alkyl, lower alkoxy, hydroxy, lower alkanoyloxy, benzoyloxy unsubstituted or substituted by one or two lower alkyl, lower alkoxy, halogen or trifluoromethyl; or $R_4$ and $R_5$ when combined and attached to the same carbon atom represent spiro-fused cyclopentyl or spiro-fused cyclohexyl; n represents the integer 6, 7, 8, 9 and 10; aryl represents phenyl or phenyl mono- or di-substituted by lower alkyl, lower alkoxy, hydroxy, halogen or trifluoromethyl; tautomers thereof; or a pharmaceutically acceptable salt thereof, the term "lower" defines such with up to and including 7 carbon atoms.

Specific distinct embodiments of the invention relate to the compounds of the formula II wherein n is either 6, 7, 8, 9 or 10; and $R_1$, $R_4$ and $R_5$ have meaning as defined herein. Preferred are the compounds wherein n represents either 6, 7 or 8, advantageously those wherein n represents 6.

Particularly preferred are the compounds of formula II wherein n has meaning as defined above; $R_1$ represents phenyl, or phenyl mono- or di-substituted by lower alkyl, lower alkoxy, halogen or trifluoromethyl; $R_4$ and $R_5$ represent hydrogen or lower alkyl; tautomers thereof; and pharmaceutically acceptable salts thereof the term "lower" defines such with up to and including 7 carbon atoms.

Further preferred are said above compounds of formula II wherein n represents the integers as defined above; $R_1$ represents phenyl or phenyl mono-substituted by lower alkyl, lower alkoxy or halogen; $R_4$ and $R_5$ represent hydrogen or lower alkyl; tautomers thereof; and pharmaceutically acceptable salts thereof the term "lower" defines such with up to and including 7 carbon atoms.

A particular embodiment relates to said compounds of formula II wherein $R_1$ represents phenyl mono-substituted at the para position by lower alkyl, lower alkoxy or halogen; and $R_4$ and $R_5$ represent hydrogen; tautomers thereof; and pharmaceutically acceptable salts thereof; which are predominantly benzodiazepine receptor agonists the term "lower" defines such with up to and including 7 carbon atoms.

Another particular embodiment relates to the compounds of formula II wherein $R_1$ represents 2-pyridyl; $R_4$ and $R_5$ represent hydrogen; n represents an integer as defined above; tautomers thereof; and pharmaceutically acceptable salts thereof, which are predominantly benzodiazepine receptor antagonists the term "lower" defines such with up to and including 7 carbon atoms.

The general definitions used herein have the following meaning within the scope of the present invention, including intermediates and starting materials.

The term "lower" referred to above and hereinafter in connection with organic radicals or compounds respectively defines as given before such with up to and including 7, preferably up and including 4 and advantageously one or two carbon atoms.

Halogen is preferably fluoro or chloro, but may also be bromo or iodo.

A lower alkyl group or such present in said lower alkoxy, or other alkylated groups, is above all methyl, but also ethyl, n- or i-(propyl, butyl, pentyl, hexyl or heptyl), e.g. 2-methylpropyl or 3-methylbutyl.

Pyridyl represents 2-, 3- or 4-pyridyl, advantageously 2-pyridyl.

Quinolyl represents preferably 2-, 3- or 4-quinolyl advantageously 3-quinolyl.

Isoquinolyl represents preferably 1-, 3- or 4-isoquinolyl, advantageously 1-isoquinolyl.

Pyrimidyl represents 2-, 4- or 5-pyrimidyl, preferably 2- or 5-pyrimidyl.

Thiazolyl represents preferably 2-thiazolyl.

A lower alkylidene group (with double bond exocyclic to the ring) represents preferably methylidene or ethylidene.

Aryl unless specified otherwise represents preferably phenyl or phenyl mono- or di-substituted by lower alkyl, lower alkoxy, hydroxy, acyloxy, halogen or trifluoromethyl.

Lower alkanoyloxy is preferably acetoxy or propionyloxy. Aroyloxy is preferably benzoyloxy or benzoyloxy substituted on the benzene ring by one or two of lower alkyl, lower alkoxy, halogen or trifluoromethyl.

The compounds of the invention wherein $R_3$ and $R_3'$ are hydrogen may be represented by either of the tautomeric structures IA or IB, preferably structure IA; furthermore said 3-oxo compounds, e.g. of formula II may, under certain conditions, also exist as the 3-hydroxy (enol) tautomers; all of these tautomers are within the scope for the present invention. Said compounds form, especially in the form of the 3-hydroxy compounds, salts with strong bases, and the salts are preferably alkali metal, e.g. sodium or potassium salts of the 1- or 5-unsubstituted compounds ($R_3$ and $R_3'$ = H).

Furthermore compounds of formula IA or IB, form acid addition salts, which are preferably such of pharmaceutically acceptable inorganic or organic acids, such as strong mineral acids, for example hydrohalic, e.g. hydrochloric or hydrobromic acid; sulfuric, phosphoric or nitric acid; aliphatic or aromatic carboxylic or sulfonic acids, e.g. acetic, propionic, succinic, glycolic, lactic, malic, tartaric, gluconic, citric, maleic, fumaric, hydroxymaleic, pyruvic, phenylacetic, benzoic, 4-aminobenzoic, anthranilic, 4-hydroxybenzoic, salicylic, 4-aminosalicylic, pamoic, nicotinic, methanesulfonic, ethanesulfonic, hydroxyethanesulfonic, benzenesulfonic, p-toluenesulfonic, naphthalenesulfonic, sulfanilic, cyclohexylsulfamic acid; or ascorbic acid.

The compounds of the invention exhibit valuable pharmacological properties, e.g. nervous system regulatory effects, by inter alia modulating the benzodiazepine receptor activity in mammals. The compounds are thus useful for the treatment of nervous system diseases, e.g. those responsive to benzodiazepine receptor modulation.

The foregoing attributes render compounds of this invention particularly useful when administered, alone or in combination, to mammals for the treatment of e.g. nervous system disorders such as anxiety and convulsive conditions (epilepsy) for compounds which are predominantly benzodiazepine receptor agonists, or as enhancers of cognitive performance and of vigilance, as somnolytics, as appetite suppressants, as antagonists (antidotes) of the effects of benzodiazepine drug overdose on the central nervous system or as antagonists of the sedative effects of alcohol and benzodiazepine drugs in combination, for compounds which are predominantly benzodiazepine receptor antagonists/inverse agonists.

The compounds of the invention bind to the benzodiazepine receptor and exhibit e.g. anxiolytic and/or anticonvulsant effects, or antagonism of the effects of benzodiazepine drugs. Said effects are demonstrable by in vitro and in vivo tests, using advantageously mammals, e.g. mice, rats, or monkeys, as test objects. Said compounds can be applied to them enterally or parenterally, advantageously orally, or subcutaneously, intravenously or intraperitoneally, for example, within gelatin capsules or in the form of aqueous solutions or suspensions respectively. The applied dosage may range between about 0.1 and 100 mg/kg/day, preferably between about 0.5 and 30 mg/kg/day, advantageously between about 1 and 25 mg/kg/day. The applied dosage in vitro may range between about $10^{-5}$ and $10^{-10}$ M concentration, preferably between about $10^{-7}$ and $10^{-9}$ M.

The benzodiazepine receptor binding properties indicative of the nervous system regulatory activity of said new compounds are determined in the receptor binding assay in vitro, e.g. similarly to that in Nature 266, 732 (1977) or Proc.Nat.Acad.Sci. USA 74, 3805 (1977). When tritiated flunitrazepam is used, the interaction of other drugs with said receptor can be readily assessed thus: Synaptosnal membranes from rat fore-brain are incubated at 0-5° for 30 minutes with 0.5 nM tritiated flunitrazepam and various concentrations of the test substance in a buffer medium maintained at pH 7.5. Solutions of the various concentrations of test substance are prepared by dilution of a 4.2 mM stock solution in dimethyl-acetamide-ethanol(1:10) with 50 mM pH 7.5 Tris-HCl buffer. The membranes, containing the receptors with various amounts of tritiated flunitrazepam, are filtered onto glass fiber filters, which are then analyzed in a liquid scintillation counter. the concentration of the compounds of this invention, required to inhibit the specific binding of 0.5 nM of tritiated flunitrazepam by 50 %, i.e. the $IC_{50}$, is determined graphically.

In vivo benzodiazepine receptor binding is determined essentially as described in Eur.J.Pharmacol. 48, 213 (1978) and Nature 275, 551 (1978).

Test compounds in a corn starch vehicle are administered orally or intraperitoneally to mice or rats. Thirty minutes later, $^{3}$H-flunitrazepam (2 nmoles/kg in saline) is injected into the tail vein, and the animals are sacrificed 20 minutes after injection of the flunitrazepam. The brains are then assayed by determining radioactivity in a liquid scintillation counter for binding of the radioligand to the receptors. A decrease in the binding of $^{3}$H-flunitrazepam in the drug-treated animals (as compared with the binding observed in animals treated with vehicle alone) is indicative of benzodiazepine receptor binding by the test compound.

Anxiolytic effects are observed, for example, by measuring the antagonism of pentylenetetrazol discriminative stimuli in the rat and according to the Cook-Davidson conflict procedure, using male Wistar rats, e.g. as described in Drug Development Research 6, 313-325 (1985).

Anticonvulsant effects are observed, for example in the standard Metrazole (pentylenetetrazole) and audiogenic seizure tests in the rat for assessing anticonvulsant activity, e.g. described in Drug Development Research 6, 313-325 (1985).

Benzodiazepine antagonism can be determined by measuring by the antagonism of the anticonvulsant activity of diazepam in the rat Metrazole model, or by measuring the antagonism of diazepam in the rat rotorod procedure, e.g. as described in Drug Development Research 6, 313-325 (1985).

Inverse agonist activity can be determined e.g. by measuring the potentiation of metrazole in the rat metrazole model.

The pharmacological agonist and/or antagonist/inverse agonist profile of the benzodiazepine receptor modulators of the invention can also be determined by measuring their effect in a rat brain membrane preparation on the displacement of $^{3}$H-flunitrazepam in the presence or absence of gamma-aminobutric acid (GABA), on the enhancement of $^{3}$H-muscimol binding by stazolate, or on the binding of $^{35}$S-butyl bicyclophosphorothionate (TBPS), e.g. as described in J.Pharmacol.Exp.Prap. 231, 572 (1984).

The compounds of the invention which bind to the benzodiazepine receptor and demonstrate a benzodiazepine agonist profile are most useful as anxiolytic and as anticonvulsant agents for the treatment of anxiety and convulsive disorders, particularly petit mal epilepsy. Illustrative thereof are the compounds of examples 1 and 2b.

Compounds of the invention which demonstrate a benzodiazepine agonist profile exhibit anxiolytic activity, e.g. in the Cook-Davidson conflict procedure, while being essentially free of effects such as sedation, tolerance, interaction with alcohol, and muscle relaxation seen with classical anxiolytic agents such as diazepam.

Sedative effects are determined e.g. in the standard rotorod performance test and alcohol interaction is determined e.g. in the rotorod alcohol interaction test in the rat.

Compounds of the invention which are benzodiazepine agonists and demonstrate anxiolytic properties, e.g. as determined in the Cook-Davidson model, also do not generalize to the discrimination test in the rat which is carried out according to Drug Development Research 6, 313-325 (1985) and in which test compounds are administered orally. Illustrative thereof is the compound of example 1.

The diazepam discrimination test, can also be used to determine benzodiazepine antagonist properties.

The compounds of the invention which bind to the benzodiazepine receptor and demonstrate a benzodiazepine antagonist/inverse agonist profile are most useful as somnolytics, as enhancers of cognitive performance and vigilance, and as appetite suppressants for the treatment of e.g. depression and obesity.

Accordingly, the compounds of the invention are useful nervous system active agents, e.g. as benzodiazepine receptor modulators, for example in the treatment or management of nervous systems disorders in mammals responsive to said modulation. They are also useful in the preparation of other valuable products, especially of pharmacologically active pharmaceutical compositions.

The compounds of the invention, i.e. the compounds of formula IA or IB and salts, or tautomers thereof, are advantageously prepared according to the following processes:

a) reacting a compound of formula III

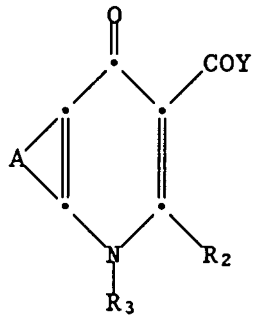

(III)

wherein A, $R_2$ and $R_3$ have meaning as previously defined, and Y is lower alkoxy; with a compound of formula IV

$R_3$'-NH-NH-$R_1$ (IV)

wherein $R_1$ has meaning as previously defined, and $R_3$' is hydrogen; or

b) reacting a compound of the formula V

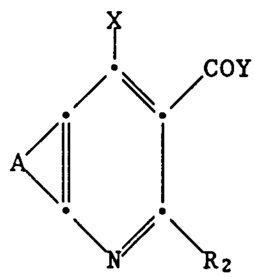

(V)

wherein A and $R_2$ have meaning as previously defined; X represents reactive etherified or esterified hydroxy; and Y represents lower alkoxy; with a compound of formula IV wherein $R_1$ has meaning as previously defined, and $R_3$' represents hydrogen or lower alkyl; or

c) cyclizing a compound of formula V above, wherein X is -N$R_3$'-NH$R_1$ and Y is lower alkoxy or hydroxy; or X is hydroxy, reactive esterified or etherified hydroxy, and Y is -N$R_1$NH$R_3$', and wherein A, $R_1$, $R_2$ and $R_3$' have meaning as previously defined; or

d) cyclizing a compound of formula V wherein X is lower alkoxyamino or azido and Y is -NH-$R_1$, and A, $R_1$ and $R_2$ have meaning as previously defined; or

e) cyclizing a compound of formula VI

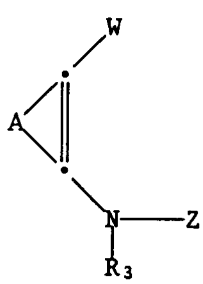

(VI)

wherein W is hydrogen, Z is

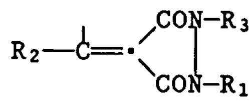

and A, $R_1$, $R_2$, $R_3$ and $R_3$' have meaning as previously defined; or

f) cyclizing a compound of formula VI above wherein W is

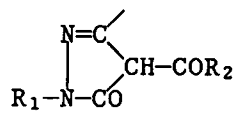

or an enamine derivative thereof, and Z is hydrogen, and A, $R_1$, $R_2$ and $R_3$ have same meaning as previously defined; or

g) cyclizing a compound of formula VI above wherein W is

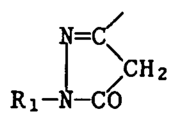

Z is $R_2CO$-, or $R_3$-N-Z is isocyano, and A, $R_1$, $R_2$ and $R_3$ have same meaning as previously defined; and if desired, converting a resulting compound of formula Ia or IB into a salt thereof or liberating a free compound from such a salt; or converting a resulting compound into another compound of the invention.

The condensation according to process a) is carried out preferably at a temperature range of about 50° to 180°C, advantageously in the presence of inert solvents such as aliphatic or aromatic hydrocarbons and ethers such as toluene, xylene, biphenyl and/or diphenyl ether, advantageously e.g. while distilling off the alkanol and water generated, or in the presence of dehydrating agents, such as molecular sieves.

The starting materials of formula III may be prepared by methods analogous to e.g. US Patent 3,429,887 and as described in the examples herein.

The starting materials of formula IV are known or are prepared by methods well known to the art.

The condensation according to process b) above is carried out with the excess or equivalent amount of a compound of formula IV advantageously and depending on the nature of the reactants at temperatures between about 50° and 200°C and preferably in an inert solvent e.g. a lower alkanol such as amyl alcohol, n-butyl alcohol or ethanol, an amide such as dimethylformamide or N-methyl-pyrolidinone, an aliphatic or aromatic hydrocarbon such as toluene, xylene or biphenyl, an aromatic ether such as diphenyl ether or mixtures thereof.

The starting materials of formula V may be prepared by methods analogous to US Patent 3,786,043 and as described in the examples herein.

In starting materials of formula V and VIII (below), when X represents reactive esterified hydroxy said group is preferably halogen such as chloro or bromo, or lower alkanesulfonyloxy such as methanesulfonyloxy, or when X represents reactive etherified hydroxy said group is preferably lower akoxy such as methoxy, or aryloxy such as phenoxy.

The ring closure of compounds of formula V according to process c) is carried out preferably at a temperature range of about 50° to 200°C, advantageously in the presence of inert solvents such as aliphatic or aromatic hydrocarbons, such as toluene, xylene or biphenyl, ethers such as diphenyl ether, alkanols such as n-butanol, with or without a base (such as an alkali metal alkoxide, e.g. sodium ethoxide), a dehydrating agent (such as molecular sieves) or a condensing agent (such as N-ethoxy-carbonyl-2-ethoxy-1,2-dihydroquinoline), depending on the nature of X and Y.

Advantageously a condensing agent or dehydrating agent is used for the ring closure of compounds of formula V wherein Y represents hydroxy.

The starting materials for process c) of formula V, wherein X is $-NR_3'-NHR_1$ and Y is lower alkoxy or hydroxy, may be obtained by condensation of a compound of formula V, wherein X represents reactive etherified or esterified hydroxy and Y represents lower alkoxy, with a hydrazine of formula IV, wherein $R_1$

and $R_3$' are as previously defined, in an inert solvent, preferably at a temperature range of about 0° to 75°C, and hydrolysis if so required.

The hydrazide starting materials of formula V wherein X is hydroxy, esterified or etherified hydroxy and Y is $-NR_1NHR_3$', are advantageously prepared by condensing a compound of formula VIII

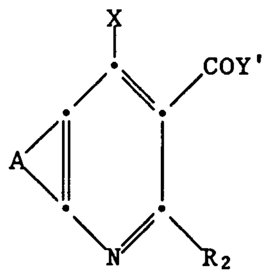

(VIII)

wherein X represents hydroxy, esterified or etherified hydroxy, COY' represents a reactive functionalized carboxy group (such as an acid halide or a mixed anhydride group) and A and $R_2$ are as previously defined, with a hydrazine of formula IV or with an $NHR_3$'-acylated derivative thereof (such as $HNR_1$-$NR_3$'-$COCF_3$) wherein $R_1$ and $R_3$' are as previously defined, and subsequently deacylating the resulting acyl-substituted hydrazide.

A preferred starting material of formula VIII is the appropriately ring-fused and substituted compound of formula VIII wherein X and Y' represent chloro.

The ring closure of compounds of formula V according to process d) is preferably carried out by heating them to temperatures between about 120° and 300°C, preferably between 200° and 250°C, advantageously also in the presence of above-cited inert solvents, e.g. eutectic diphenyl ether-biphenyl.

The starting materials for process d) of formula V are preferably obtained by condensing 4-halo-cycloalka[b]-pyridine-3-carboxylic acid halides with an $R_1$-amine, and subsequently with a O-lower alkyl-hydroxylamine (a lower alkoxyamine) or an alkali metal azide.

The starting materials for process d) of formula V may also be prepared from the compounds of formula IX

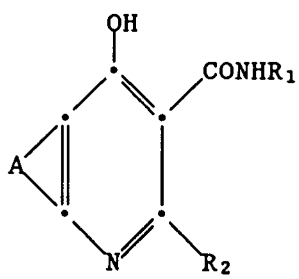

(IX)

or tautomers thereof, wherein A, $R_1$ and $R_2$ have meaning as previously defined for the compounds of formula IA, by derivatization first to the corresponding 4-halo-cycloalka-[b]-pyridine derivatives and subsequently to the compounds of formula V wherein X is lower alkoxyamino or azido, and Y is $-NHR_1$.

The compounds of formula IX are in turn prepared e.g. by condensation of the compounds of formula V wherein A and $R_2$ have meaning as defined above, X represents hydroxy and Y represents lower alkoxy, with an amine $R_1$-$NH_2$ wherein $R_1$ has meaning as previously defined above, under aminolysis conditions well-known in the art, preferably in the presence of a base such as triisobutylaluminium, advantageously at about room temperature, in an inert solvent such as tetrahydrofuran, methylene chloride or toluene.

The compounds of formula IX, alkali metal salts and acid-addition salts thereof derived from pharmaceutically acceptable inorganic or organic acids as given above in connection with acid addition salts of compounds of formula IA or IB, exhibit benzodiazepine-receptor modulating activity and are thus useful for the treatment of nervous system diseases, such as anxiety and convulsive conditions. Benzodiazepine receptor binding, anxiolytic, anticonvulsant activity or benzodiazepine antagonist/inverse agonist or agonist profile activity are determined in vitro and in vivo using methodology as described above for the compounds of formula IA or IB.

For the in vitro receptor binding assay procedures, the compounds of formula IX are applied at a concentration ranging from about $10^{-5}$ M to about $10^{-9}$ M. For in vivo tests, the applied dosage may range between about 0.1 and 200 mg/kg/day, preferably between about 0.5 and 50 mg/kg/day, advantageously between about 1 and 30 mg/kg/day.

Preferred are the compounds of formula IX wherein A together with the two carbon atoms to which it is attached represents a fused ring in which A represents hexamethylene, heptamethylene, octamethylene, nonamethylene or decamethylene, each unsubstituted or mono- or di-substituted on carbon atoms within A by lower alkyl, hydroxy, acyloxy, oxo, lower alkoxy, aryl, aryl-lower alkyl or aryl-lower alkoxy; $R_1$ represents lower alkyl, phenyl or phenyl mono- or di-substituted by lower alkyl, lower alkoxy, halogen or trifluoromethyl; or $R_1$ represents pyridyl, quinolyl, isoquinolyl, pyrimidyl or thiazolyl; and $R_2$ represents hydrogen; and pharmaceutically acceptable salts thereof.

The cyclization of compounds of formula VI according to process e) is preferably carried out with strong aprotic condensation agents, such as polyphosphoric acid lower alkyl esters, advantageously in the presence of inert solvents such as halogenated aliphatic hydrocarbons, e.g. 1,1,2,2-tetrachlorethane.

The starting materials for process e) of formula VI as defined above for process e) can be prepared according to known methods, e.g. by condensing a 1-aryl-pyrazolidin-3,5-dione with a starting material of formula VI wherein W is hydrogen and Z is formyl. Said N-formylenamine derivatives useful as starting materials are prepared e.g. as described in Compt.Rend. 264, 333 (1967).

The cyclization of compounds of formula VI according to process f) is preferably carried out in the presence conventional molecular sieves, and/or a catalytic amount of acid, e.g. hydrogen chloride.

A modification of process f) involves the cyclization of a compound of formula VI wherein W represents the enamine grouping

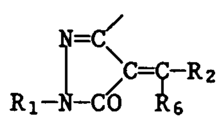

wherein $R_6$ represents e.g. di-lower alkylamino, piperidino or morpholino; A, $R_1$, $R_2$ and $R_3$ have meaning as previously defined; and Z represents hydrogen.

A further modification of process f) involves the condensation of a compound of formula VI wherein $R_3$ and Z together with the nitrogen to which they are attached represent e.g. di-lower alkylamino, piperidino or morpholino, and W represents the enamine grouping cited just above, with the amine $R_3$-$NH_2$ preferably in the presence of an acid-addition salt thereof such as the acetic acid salt, preferably in an inert solvent such as ethanol.

The appropriate 3-substituted-pyrazol-5-one starting materials of formula VI as defined for process f) can be prepared analogous to the process described in Latvijas PSR Zinatku Akad. Vestis, Khim.Ser. 1965 - (5) 587-92, using the suitable intermediates as required for said compounds.

The cyclization of compounds of formula VI according to process g) is preferably carried out under basic conditions, e.g. in the presence of alkali metal hydroxides, or tertiary organic amines, such as tri-lower alkylamines.

The starting materials of formula VI as defined for process g) above may be prepared by e.g. dehalogenation of a compound of the formula X

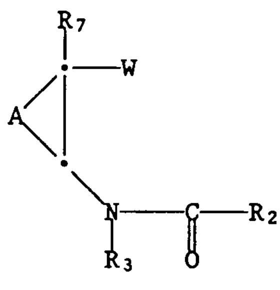

(X)

wherein $R_7$ represents halogen, advantageously bromo; and A, W, $R_2$ and $R_3$ have meaning as defined above.

The above intermediates of formula X may in turn be prepared by photochemical addition of e.g. N-bromo-formamide or N-bromo-lower alkylcarboxamide [as described in Can.J.Chem. 59, 431 (1981)] to the corresponding (α,β-unsaturated carboxcyclic)-substituted β-ketoacetic acid lower alkyl ester, followed by condensation with $R_1$-$NHNH_2$.

The intermediates of formula VI for process g) wherein $R_2$ and $R_3$ are hydrogen, may, if desired, be dehydrated to the isonitriles with phosphorus halides or phosphorus oxyhalides.

The compounds of the invention so obtained can be converted into other compounds of formula IA or IB according to known methods.

For example compounds of formula IA or IB with $R_3$ or $R_3'$ = H can be 1-substituted with reactive esters of $R_3$-OH, e.g. such of hydrohalic, aliphatic or aromatic sulfonic acids, such as $R_3$-(halides, sulfates, aliphatic or aromatic sulfonates), e.g. methyl iodide, dimethyl sulfate, methyl mesylate or tosylate, in order to yield the 1-substituted compounds of formula IB. Those of formula IA are similarly obtained from the corresponding alkali metal salts, e.g. the sodium salt, whereby 5-substitution occurs. The metal derivative intermediates are obtained by metallation with reactive organometallic agents such as lithium diisopropylamide, with alkali metal alkoxides such as sodium methoxide, or thallous ethoxide, or alkali metal hydrides such as sodium or potassium hydride.

The compounds with an oxo function within A (ketones) may be converted to the corresponding compounds with a hydroxy function within A (alcohols), e.g. of formula II wherein $R_4$ or $R_5$ represents hydroxy, by reduction, e.g. with a metal hydride reducing agent such as sodium borohydride. Said ketones may also be converted to the tertiary alcohols, e.g. to the compounds of formula III wherein $R_4$ and $R_5$ are on the same carbon atom and represent e.g. lower alkyl and hydroxy, by treatment with e.g. a Grignard reagent such as a lower alkyl magnesium halide.

The compounds with a hydroxy function within A, e.g. the compounds of formula II wherein $R_4$ or $R_5$ represents hydroxy, may in turn be converted to the corresponding compounds with an oxo function within A, by treatment with an oxidizing agent such as pyridinium chlorochromate. Said hydroxy compounds may also be converted to the corresponding acyloxy substituted compounds (esters) by esterification methods well-known in the art.

Finally, the compounds of the invention are either obtained in the free form, or as a salt thereof whenever applicable. Any resulting free base can be converted into a corresponding acid addition salt, preferably with the use of pharmaceutically acceptable acid or anion exchange preparation, or any resulting salt can be converted into the corresponding free base, for example, with the use of a stronger base, such as a metal or ammonium hydroxide or a basic salt, e.g. an alkali metal hydroxide or carbonate, or a cation exchange preparation. Said acid addition salts are preferably such of pharmaceutically acceptable inorganic or organic acids described previously.

Compounds of formula IA or IB with $R_3$ or $R_3'$ being hydrogen can also be converted into the corresponding metal salts by e.g. treatment with the alkaline or alkaline earth metal hydroxides or carbonates.

These and other salts, for example, the picrates, can also be used for purification of the bases obtained, the bases are converted into salts, the salts are separated and the bases liberated from the salts.

In view of the close relationship between the free compounds and the compounds in the form of their salts, whenever a compound is referred to in this context, a corresponding salt is also intended, provided such is possible or appropriate under the circumstances.

The compounds including their salts, can also be obtained in the form of their hydrates or include other solvents used for crystallization.

In case mixtures of isomers of any of the above compounds are obtained, these can be separated into the single isomers by methods in themselves known, e.g. by fractional distillation, crystallization and/or chromatography. Any racemic products can be resolved into the individual optical antipodes.

Any basic racemic products or intermediates can be resolved into the optical antipodes, for example, by separation of diastereomeric salts thereof, e.g., by the fractional crystallization of d- or 1-(tartrate, dibenzoyltartrate, mandelate or camphorsulfonate) salts.

Any acidic racemic products of intermediates can be resolved by separation of e.g. the d- and 1-(methylbenzylamine, cinchonidine, cinchonine, quinine, quinidine, ephedrine, dehydroabiethylamine, brucine or strychnine)-salts.

The above-mentioned reactions are carried out according to standard methods, in the presence or absence of diluents, preferably such as are inert to the reagents and are solvents thereof, of catalysts, condensing or said other agents respectively and/or inert atmospheres, at low temperatures, room temperature or elevated temperatures, preferably near the boiling point of the solvents used, at atmospheric or superatmospheric pressure.

The invention further includes any variant of the present processes, in which an intermediate product obtainable at any stage thereof is used as starting material and the remaining steps are carried out, or the process is discontinued at any stage thereof, or in which the starting materials are formed under the reaction conditions, or which the reaction components are used in the form of their salts or pure isomers. Mainly those starting materials should be used in said reactions, that lead to the formation of those compounds, indicated above as being especially valuable.

In starting compounds and intermediates which are converted to the compounds of the invention in a manner described herein, functional groups present, such as carbonyl (formyl or keto), carboxy, amino and hydroxy groups, may be protected by conventional protecting groups that are common in preparative organic chemistry. Protected carbonyl, carboxy, amino and hydroxy groups are those that can be converted under mild conditions into free carbonyl, carboxy, amino and hydroxy groups without the molecular framework being destroyed or other undesired side reactions taking place. The need and choice of protecting groups for a particular reaction is known to those skilled in the art and depends on the nature of the functional group to be protected (carbonyl group, carboxy group, amino group etc.), the structure and stability of the molecule of which the substitutent is a part, and the reaction conditions.

Well-known protecting groups that meet these conditions and their introduction and removal are described, for example, in J.F.K. McOmie, "Protective Groups in Organic Chemistry", Plenum Press, London, New York 1973, T.W. Greene, "Protective Groups in Organic Synthesis", Wiley, New York 1984, and also in "The Peptides", Vol. I, Schroeder and Luebke, Academic Press, London, New York 1965, as well as in Houben-Weyl, "Methoden der Organischen Chemie", Vol. 15/1, George Thieme Verlag, Stuttgart, 1974.

The pharmacologically active compounds of the invention are useful in the manufacture of pharmaceutical compositions comprising an effective amount thereof in conjunction or admixture with excipients suitable for either enteral, parenteral or transdermal application.

Preferred are tablets and gelatin capsules comprising the active ingredient together with a) diluents, e.g. lactose, dextrose, sucrose, mannitol, sorbitol, cellulose and/or glycine; b) lubricants, e.g. silica, talcum, atearic acid, its magnesium or calcium salt and/or polyethyleneglycol; for tablets, also c) binders, e.g. magnesium aluminium silicate, starch paste, gelatin, tragacanth, methylcellulose, sodium carboxymethylcellulose and/or polyvinyl-pyrrolidone; if desired, d) disintegrants, e.g. starches, agar, alginic acid or its sodium salt, or effervescent mixtures; and/or e) absorbents, colorants, flavors and sweeteners. Injectable compositions are preferably aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salt for regulating the osmotic pressure and/or buffers. In addition, they may also contain other therapeutically valuable substances. Said compositions are prepared according to conventional mixing, granulating or coating methods, respectively, and contain about 0.1 to 75 %, preferably about 1 to 50 %, of the active ingredient. Suitable formulations for transdermal application include an effective amount of a pharmacologically active compound of the invention with carrier. Advantageous carriers include absorbable pharmacologically acceptable solvents to assist passage through the skin of the host. Characteristically, transdermal devices are in the form of a bandage comprising a backing member, a reservoir containing the compound, optionally with carriers, optionally a rate controlling barrier to deliver the compound to the skin of the host at a controlled and predetermined rate over a prolonged period of time, and means to secure the device to the skin.

The invention also relates to a method of treatment of disorders in mammals responsive to an benzodiazepine receptor agonist or antagonist/inverse agonist advantageously to the method of treatment of nervous system disorders responsive to the action of a benzodiazepine receptor agonist, using an effective amount of a compound of the invention e.g. of formula II, or pharmaceutically acceptable salts of such compounds, as pharmacologically active substances, preferably in the form of above-cited pharmaceutical compositions. The dosage of active compound administered is dependent on the species of warm-blooded animal (mammal), the body weight, age and individual condition, and on the form of administration.

A unit dosage for a mammal of about 50 to 70 kg may contain between about 10 and 200 mg of the active ingredient.

The following examples are intended to illustrate the invention and are not to be construed as being limitations thereon. Temperatures are given in degrees Centigrade. If not mentioned otherwise, all evaporations are performed under reduced pressure, preferably between about 15 and 100 mm Hg.

Example 1

A mixture of 2.24 g of ethyl 4-chloro-5,6,7,8,9,10-hexahydrocycloocta[b]pyridine-3-carboxylate and 1.16 g of 4-methoxyphenylhydrazine is stirred and heated at reflux under a nitrogen atmosphere for 20 hours in 20 ml of n-butanol. The solvent is then evaporated under reduced pressure and the residual material stirred with 20 ml of 1N sodium hydroxide, ether, and water. The layers are separated, the aqueous phase is extracted with ether and then treated with an aqueous solution of 1.07 g of ammonium chloride. The precipitate is filtered off and washed with water to give 2-(4-methoxyphenyl)-2,3,6,7,8,9,10,11-octahydrocycloocta[b]pyrazolo[3,4-d]pyridin-3(5H)-one, m.p. 275-277 ° C, the compound of formula II wherein n represents the integer 6, $R_4$ and $R_5$ represent hydrogen, and $R_1$ represents p-methoxyphenyl. Further purification gives m.p. 283-285 ° C.

The starting material is prepared as follows:
A solution of 139 g of cyclooctanone, 103 g of diethyl aminoethylenemalonate, and 7 g of dichloroacetic acid in 500 ml toluene is refluxed for 60 hours with a water separator under nitrogen atmosphere, then evaporated to dryness. The residual oil is triturated with 1600 ml heptane and the heptane extract evaporated to dryness. The dried material is purified by flash chromatography on a silica gel column with 2 % ethyl acetate in methylene chloride as eluent to give a mixture of cyclooctanone and diethyl N-(1-cyclooctenyl)-aminomethylenemalonate.

A solution of 85 g of the mixture of cyclooctanone and diethyl N-(1-cyclooctenyl)-aminomethylenemalonate in 20 ml of Dowtherm® (an eutectic mixture of diphenyl ether and biphenyl) is added to 380 ml of Dowtherm® at 250-255 ° C under nitrogen. The distillate is collected in a water separator. After 0.5 hour the mixture is cooled to room temperature and solvent is removed under reduced pressure. The residual solid is triturated with ether to give ethyl 5,6,7,8,9,10-hexahydro-4-hydroxycycloocta-[b]pyridine-3-carboxylate, m.p. 219-222 ° C.

A solution of 5 g of ethyl 5,6,7,8,9,10-hexahydro-4-hydroxycycloocta[b]pyridine-3-carboxylate in 20 ml of phosphorus oxychloride is refluxed for one hour and evaporated to dryness under reduced pressure. A solution of the residue in methylene chloride is treated with ice and water, and basified with 10 N sodium hydroxide. The layers are separated, the aqueous phase reextracted with methylene chloride, and the combined organic layers dried with magnesium sulfate, filtered, and solvent removed at reduced pressure to obtain ethyl 5,6,7,8,9,10-hexahydro-4-chlorocycloocta[b]pyridine-3-carboxylateas an oil which solidifies on standing, m.p. 33-36 ° C.

Example 2

a) Reaction of ethyl 5,6,7,8,9,10-hexahydro-4-chlorocycloocta[b]pyridine-3-carboxylate with phenylhydrazine according to the procedure analogous to that described in example 1 yields 2,3,6,7,8,9,10,11-octahydro-2-phenylcycloocta[b]pyrazolo[3,4-d]pyridin-3(5H)-one, m.p. 310-312 ° C.
b) Similarly prepared is 2-(4-chlorophenyl)-2,3,6,7,8,9,10,11-octahydrocycloocta[b]pyrazolo[3,4-d]pyridin-3(5H)-one, m.p. 309-311 ° C.

Example 3

Octahydrocycloocta[b]pyrazolo[3,4-d]pyridin-3(5H)-one derivatives of formula II, wherein n represents the integer 6, which can be prepared by procedures analogous to those described in example 1, using an appropriately substituted hydrazine and optionally substituted cyclooctanone as starting materials:

| Example | $R_1$ | $R_4$ | $R_5$ | m.p. |
|---|---|---|---|---|
| 3/a | 3-pyridyl | H | H | 296-300 ° C |
| 3/b | 2-thiazolyl | H | H | |
| 3/c | 2-pyrimidyl | H | H | |
| 3/d | 6-methyl-3-pyridyl | H | H | |
| 3/e | 3-quinolyl | H | H | |
| 3/f | p-fluorophenyl | H | H | 320-322 ° C |
| 3/g | p-methoxyphenyl | 9-$CH_3$ | H | |
| 3/h | p-chlorophenyl | 7-$CH_3$ | 11-$CH_3$ | |
| 3/i | 2-pyridyl | H | H | 180-188 ° C |
| 3/j | p-ethylphenyl | H | H | 296-300 ° C |
| 3/k | p-ethoxyphenyl | H | H | 299-301 ° C |
| 3/l | 4-pyridyl | H | H | > 320 ° C |

5-Methylcyclooctanone, the starting ketone for the preparation of compound 3/g is described in Bull.Soc.Chim. France 1972 (5), 2024-7.

3,7-Dimethylcyclooctanone, the starting ketone for the preparation of compound 3/h is described in Zh.Org.Khim. 13 (9), 1984-7 (1977).

Example 4

Decahydrocyclonona[b]pyrazolo[3,4-d]pyridin-3-one derivatives of formula II wherein n represents the integer 7 which can be prepared by procedures analogous to those described in example 1, using an appropriately substituted hydrazine and optionally substituted cyclononanone as starting materials:

| Example | $R_1$ | $R_4$ | $R_5$ |
|---|---|---|---|
| 4/a | 3-pyridyl | H | H |
| 4/b | 2-thiazolyl | H | H |
| 4/c | 2-pyrimidyl | H | H |
| 4/d | 6-methyl-3-pyridyl | H | H |
| 4/e | 3-quinolyl | H | H |
| 4/f | p-methoxyphenyl | H | H |
| 4/g | phenyl | H | H |
| 4/h | p-chlorophenyl | H | H |
| 4/i | p-tolyl | H | H |

Example 5

Decahydrocyclodeca[b]pyrazolo[3,4-d]pyridin-3(5H)-one derivatives of formula II wherein n represents the integer 8 which can be prepared by procedures analogous to those described in example 1 using an appropriately substituted hydrazine and optionally substituted cyclodecanone as starting materials:

| Example | $R_1$ | $R_4$ | $R_5$ | m.p. |
|---|---|---|---|---|
| 5/a | 3-pyridyl | H | H | |
| 5/b | 2-thiazolyl | H | H | |
| 5/c | 2-pyrimidyl | H | H | |
| 5/d | 6-methyl-3-pyridyl | H | H | |
| 5/e | 3-quinolyl | H | H | |
| 5/f | 2-pyridyl | H | H | |
| 5/g | p-methoxyphenyl | H | H | 143-150°C |
| 5/h | p-fluorophenyl | H | H | |
| 5/i | phenyl | H | H | |
| 5/j | p-chlorophenyl | H | H | |
| 5/k | p-methoxyphenyl | 10-$OCH_3$ | H | |

6-Methoxycyclodecanone, the ketone starting material for compound 5/k is described in J.Org.Chem. 47, 2685-2690 (1982).

Example 6

Dodecahydrocycloundeca[b]pyrazolo[3,4-d]pyridine-3-one derivatives of formula II wherein n represents the integer 9, $R_4$ and $R_5$ represent hydrogen, which can be prepared by procedures analogous to those described in example 1 using an appropriately substituted hydrazine and cycloundecanone as starting materials:

| Example | $R_1$ |
|---|---|
| 6/a | 3-pyridyl |
| 6/b | p-fluorophenyl |
| 6/c | 2-pyrimidyl |
| 6/d | 3-quinolyl |
| 6/e | p-methoxyphenyl |
| 6/f | m-fluorophenyl |
| 6/g | 2-pyridyl |
| 6/h | phenyl |
| 6/i | p-chlorophenyl |

Example 7

Dodecahydrocyclododeca[b]pyrazolo[3,4-d]pyridin-3(5H)-one derivatives of formula II when n represents the integer 10, which can be prepared by procedures analogous to those described in example 1 using an appropriately substituted hydrazine and an optionally substituted cyclododecanone as starting materials:

| Example | $R_1$ | $R_4$ | $R_5$ | m.p. |
|---|---|---|---|---|
| 7/a | 3-pyridyl | H | H | |
| 7/b | p-fluorophenyl | H | H | |
| 7/c | 2-pyrimidyl | H | H | |
| 7/d | 3-quinolyl | H | H | |
| 7/e | p-methoxyphenyl | H | H | 303-307°C |
| 7/f | m-fluorophenyl | H | H | |
| 7/g | 2-pyridyl | H | H | |
| 7/h | phenyl | H | H | |
| 7/i | p-chlorophenyl | H | H | 289-292°C |
| 7/j | p-methoxyphenyl | 11-$OCOCH_3$ | H | |

7-Acetoxycyclododecanone, the starting ketone for preparing compound 7/j is described in

J.Chem.Soc.Chem.Commun. 1978 (9), 413-414.

The starting ethyl 4-chlorodecahydrocyclododeca[b]pyridine-3-carboxylate for compounds 7/a-7/i wherein $R_4$ and $R_5$ represent hydrogen is prepared from cyclododecanone as follows:
A solution of 50 g cyclododecanone, 103 g of diethyl aminomethylenemalonate, and 1.8 g of dichloroacetic acid in 300 ml toluene is refluxed for 72 hours with a water separator under nitrogen, then evaporated to dryness. The residual oil is triturated with heptane and the heptane extract evaporated to dryness. The dried material is purified by flash chromatography on a silica gel column with methylene chloride as eluent to give a mixture of cyclododecanone and diethyl N-(1-cyclododecenyl)-aminomethylenemalonate.

A solution of 10 g of the mixture of cyclododecanone and diethyl N-(1-cyclododecenyl)-aminomethylenemalonate in 25 ml Dowtherm® is added to 200 ml Dowtherm® at 250° under nitrogen, collecting distillate in a water separator. After heating for 0.5 hour the mixture is cooled to room temperature and the solvent distilled off under reduced pressure. The residual solid is triturated with ether and dried to give ethyl 5,6,7,8,9,10,11,12,13,14-decahydro-4-hydroxycyclododeca[b]pyridine-3-carboxylate, m.p. 182-190°C.

A solution of 2 g of ethyl 5,6,7,8,9,10,11,12,13,14-decahydro-4-hydroxycyclododeca[b]pyridine-3-carboxylate in 25 ml phosphorus oxychloride is refluxed for 2 hours and evaporated to dryness under reduced pressure. The residue is dissolved in methylene chloride, treated with ice and basified with saturated sodium carbonate solution. the layers are separated, the aqueous phase re-extracted with methylene chloride, and the combined organic layers dried with magnesium sulfate, filtered, and evaporated to dryness to obtain ethyl 4-chloro-5,6,7,8,9,10,11,12,13,14-decahydrocyclododeca[b]pyridine-3-carboxylate as an oil.

## Example 8

a) Using procedures analogous to those described in Example 1 4,4,7,7-tetramethylcyclononanone, described in Acta.Chem.Scand. 26 (3), 952-960 (1972), can be converted to 2-(4-methoxyphenyl)-2,3,5,6,7,8,9,10,11,12-decahydro-8,8,11,11-tetramethylcyclonona[b]pyrazolo[3,4-d]pyridine-3-one;
b) similarly, 4,4,8,8-tetramethyldecanone, described in Acta.Chem.Scand. 26 (3), 952-960 (1972), can be converted to 2-(4-chlorophenyl)-2,3,6,7,8,9,10,11,12,13-decahydro-8,8,12,12-tetramethylcyclodeca[b]-pyrazolo[3,4-d]pyridin-3(5H)-one.

## Example 9

A mixture of 2.00 g of ethyl 4-chloro-5,6,7,8,9,10,11,12-octahydrocyclodeca[b]pyridine-3-carboxylate and 1.12 g of 4-methoxyphenylhydrazine are stirred for eight hours in 20 ml of N-methylpyrrolidinone at 105°C. The solution is then diluted with 50 ml of water and twice extracted with ether. The combined ether fractions are extracted twice with 20 ml of 1N NaOH and the combined alkaline fractions are back-extracted with ether. The alkaline aqueous solution is then treated with an aqueous solution of 2.14 g ammonium chloride, stirred until a solid forms, the precipitate is filtered off and dried to give 2-(4-methoxyphenyl)-2,3,6,7,8,9,10,11,12,13-decahydrocyclodeca[b]pyrazolo[3,4-d]pyridin-3(5H)-one, m.p. 143-150°C, the compound of example 5/g.

The starting material is prepared as follows:
A mixture of 25.0 g of cyclodecanone, 60.7 g of diethyl aminomethylenemalonate, 1.54 g of p-toluenesulfonic acid monohydrate in 500 ml xylene is refluxed 2 weeks under argon, collecting the distillate in a Dean-Stark trap. The solution is then decanted and solvent is removed. The residual oil is triturated twice with hexane and the combined hexane extract is concentrated to yield an oil which is flash chromatographed on 600 g of silica gel using 99:1 methylene chloride ethyl acetate as eluent. A forerun containing cyclodecanone is discarded and the fractions containing the product are combined and concentrated to dryness to give diethyl N-(1-cyclodecenyl)-aminomethylenemalonate as an oil.

A solution of 46.0 g of diethyl n-(1-cyclododecenyl)-aminomethylenemalonate in 40 ml of Dowtherm® is added to one liter of Dowtherm® at 250°C under argon, and the distillate is collected in a Dean-Stark trap. After 0.5 hour the mixture is allowed to cool to room temperature and the solvent is distilled off. The residual solid is triturated with ether to give ethyl 4-hydroxy-5,6,7,8,9,10,11,12-octahydrocyclodeca[b]-pyridine-3-carboxylate, m.p. 212-230°C.

A mixture of 4.0 g of ethyl 4-hydroxy-5,6,7,8,9,10,11,12-octahydrocyclodeca[b]pyridine-3-carboxylate and 25 ml of phosphorus oxychloride is refluxed under argon for 3 hours and then evaporated to dryness under reduced pressure. A solution of the residue in methylene chloride is treated with ice and water and basified with 10N sodium hydroxide. The layers are separated, the aqueous phase is re-extracted with methylene chloride; the combined organic extract is dried with magnesium sulfate, filtered, and the solvent

is removed at reduced pressure to yield ethyl 4-chloro-5,6,7,8,9,10,11,12-octahydrocyclodeca[b]pyridine-3-carboxylate as an oil.

Example 10

Preparation of 1,000 capsules each containing 10 mg of the active ingredient:

| Formula: | |
|---|---|
| 2-(4-methoxyphenyl)-2,3,6,7,8,9,10,11-octahydrocycloocta[b]pyrazolo[3,4-d]pyridin-3-(5H)one | 10,0 g |
| Lactose | 207,0 g |
| Modified starch | 80,0 g |
| Magnesium stearate | 3,0 g |

Procedure:

All the powders are passed through a screen with openings of 0.6 mm. Then the drug substance is placed in a suitable mixer and mixed first with the magnesium stearate, then with the lactose and starch until homogeneous. Hard gelatin capsules are filled with 300 mg of said mixture each, using a capsule filling machine.

Analogously capsules are prepared, containing about 10-200 mg of the other compounds disclosed and exemplified herein.

**Claims**

**Claims for the following Contracting States : AT, BE, CH, DE, FR, GB, IT, LI, LU, NL, SE**

**1.** A compound of the formula

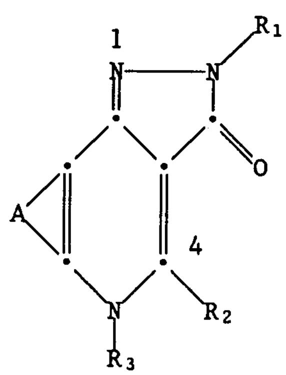

(IA)

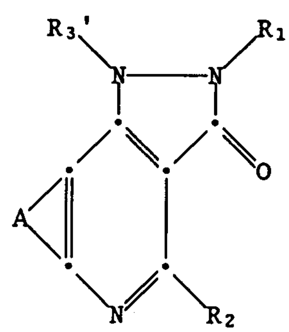

(IB)

wherein A represents an optionally substituted saturated divalent grouping which together with the two carbon atoms to which it is attached represents a fused 8, 9, 10, 11 or 12 membered carbocyclic ring selected from cycloocteno, cyclononeno, cyclodeceno, cycloundeceno and cyclododeceno; each unsubstituted or mono-, di-, tri-or tetra-substituted on carbon atoms within A by lower alkyl, lower alkylidene, $C_3$-$C_7$-cycloalkyl, hydroxy, lower alkanoyloxy, benzoyloxy unsubstituted or substituted by one or two lower alkoxy alkyl, lower alkoxy, halogen or trifluormethyl, oxo, lower alkoxy, aryl or aryl-lower alkoxy; and when disubstituted on the same carbon atom within A, said carbon atom in each ring is substituted by two lower alkyl or two aryl-lower alkyl groups, or by one lower alkyl or aryl-lower alkyl group and one group selected from hydroxy, lower alkoxy, aryl-lower alkoxy, the term aryl within the above definitions represents phenyl or phenyl mono- or disubstituted by lower alkyl, lower alkoxy, hydroxy, lower alkanoyloxy, benzoyloxy unsubstituted or substituted by one or two lower alkyl, lower alkoxy, halogen or trifluoromethyl and lower alkanoyloxy, benzoyloxy unsubstituted or substituted by one or two lower alkoxy alkyl, lower alkoxy, halogen or trifluormethyl or each disubstituted on the same carbon atom within A by straight chain alkylene of 2 to 6 carbon atoms forming with the carbon to

which the alkylene chain is attached a spiro-fused 3 to 7 membered ring; or each ring is disubstituted on adjacent carbon atoms by alkylene of 3,4 or 5 carbon atoms to form with the two adjacent carbon atoms to which said alkylene grouping is attached a fused 5-, 6-, or 7-membered ring; $R_1$ represents lower alkyl, phenyl, or phenyl mono- or disubstituted by lower alkyl, lower alkoxy, halogen or trifluoromethyl; or $R_1$ represents an aromatic heterocyclic radical selected from pyridyl, quinolyl, isoquinolyl, pyrimidyl and thiazolyl, or any said heterocyclic radical mono- or di-substituted on carbon by lower alkyl, lower alkoxy or halogen; and $R_2$, $R_3$ and $R_3$' independently represent hydrogen or lower alkyl; or a pharmaceutically acceptable salt thereof, the term "lower" defines such with up to and including 7 carbon atoms.

**2.** A compound according to claim 1 of formula IA or IB wherein A together with the two carbon atoms to which it is attached represents a fused ring selected from cycloocteno, cyclononeno, cyclodeceno, cycloundeceno and cyclododeceno in which A represents hexamethylene, heptamethylene, octamethylene, nonamethylene or decamethylene respectively; each said ring unsubstituted or mono-, di-, tri- or tetra-substituted on carbon atoms within A by lower alkyl, lower alkylidene, $C_3$-$C_7$-cycloalkyl, hydroxy, lower alkanoyloxy, benzoyloxy unsubstituted or substituted by one or two lower alkoxy alkyl, lower alkoxy, halogen or trifluormethyl, oxo, lower alkoxy, aryl, aryl-lower alkyl or aryl-lower alkoxy; and when disubstituted on the same carbon atom within A, said carbon atom in each ring is preferably substituted by two lower alkyl or two aryl-lower alkyl groups, or by one lower alkyl or aryl-lower alkyl and one group selected from hydroxy, lower alkoxy, aryl-lower alkoxy the term aryl within the above definitions represents phenyl or phenyl mono- or disubstituted by lower alkyl, lower alkoxy, hydroxy, lower alkanoyloxy, benzoyloxy unsubstituted or substituted by one or two lower alkyl, lower alkoxy, halogen or trifluoromethyl and lower alkanoyloxy, benzoyloxy unsubstituted or substituted by one or two lower alkoxy alkyl, lower alkoxy, halogen or trifluormethyl; or each ring is disubstituted on the same carbon atom within A by ethylene, propylene, butylene or pentylene forming with the carbon to which the alkylene chain is attached a spiro fused cyclopropyl, cyclobutyl, cyclopentyl or cyclohexyl ring; or each ring is disubstituted on adjacent carbon atoms by propylene or butylene to form with the two adjacent carbon atoms to which said alkylene grouping is attached a fused cyclopentane or cyclohexane ring; $R_1$, $R_2$, $R_3$ and $R_3$' have meaning as defined in claim 2; or a pharmaceutically acceptable salt thereof, the term "lower" defines and with up to and including 7 carbon atoms.

**3.** A compound according to claim 1 of the formula

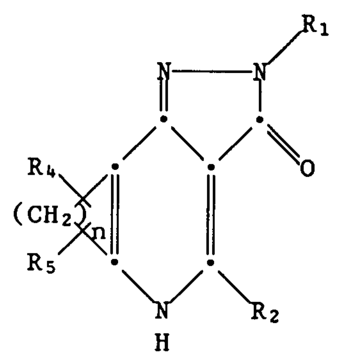

(II)

wherein $R_1$ represents lower alkyl, phenyl or phenyl mono- or di-substituted by lower alkyl, lower alkoxy, halogen or trifluoromethyl; or $R_1$ represents an aromatic heterocyclic radical selected from pyridyl, quinolyl, isoquinolyl, pyrimidyl and thiazolyl, or any said radical mono-or di-substituted by lower alkyl, lower alkoxy or halogen; $R_4$ and $R_5$ represent independently hydrogen, lower alkyl, $C_3$-$C_7$-cycloalkyl, hydroxy, lower alkanoyloxy, benzoyloxy unsubstituted or substituted by one or two lower alkoxy alkyl, lower alkoxy, halogen or trifluormethyl, lower alkoxy, aryl, aryl-lower alkyl or aryl-lower alkoxy the term aryl within the above definitions represents phenyl or phenyl mono- or disubstituted by lower alkyl, lower alkoxy, hydroxy, lower alkanoyloxy, benzoyloxy unsubstituted or substituted by one or two lower alkyl, lower alkoxy, halogen or trifluoromethyl; or $R_4$ and $R_5$ when combined and attached to the same carbon atom represent spiro-fused cyclopentyl or spiro-fused cyclohexyl; n represents the integer 6, 7, 8, 9 and 10; aryl represents phenyl or phenyl mono- or di-substituted by lower alkyl, lower alkoxy, hydroxy, halogen or trifluoromethyl; tautomers thereof; or a pharmaceutically acceptable salt

thereof, the term "lower" defines such with up to and including 7 carbon atoms.

**4.** A compound according to claim 3 wherein n represents the integer 6.

**5.** A compound according to claim 3 wherein n represents the integer 7.

**6.** A compound according to claim 3 wherein n represents the integer 8.

**7.** A compound according to claim 3 wherein n represents the integer 9.

**8.** A compound according to claim 3 wherein n represents the integer 10.

**9.** A compound according to claim 3 wherein n represents an integer as defined therein; $R_1$ represents phenyl, or phenyl mono- or di-substituted by lower alkyl, lower alkoxy, halogen or trifluormethyl; $R_4$ and $R_5$ represent hydrogen or lower alkyl; a tautomer thereof; or a pharmaceutically acceptable salt thereof, the term "lower" defines such with up to and including 7 carbon atoms.

**10.** A compound according to claim 3 wherein n represents an integer as defined therein; $R_1$ represents phenyl or phenyl monosubstituted by lower alkyl, lower alkoxy or halogen; $R_4$ and $R_5$ represent hydrogen or lower alkyl; a tautomer thereof; or a pharmaceutically acceptable salt thereof, the term "lower" defines such with up to and including 7 carbon atoms.

**11.** A compound according to claim 10 wherein $R_1$ represents phenyl mono-substituted at the para position by lower alkyl, lower alkoxy or halogen; and $R_4$ and $R_5$ represent hydrogen, a tautomer therof, or a pharmaceutically acceptable salt therof, the term "lower" defines such with up to and including 7 carbon atoms.

**12.** A compound according to claim 3 wherein n represent an integer as defined therein, $R_1$ represents 2-pyridyl, $R_4$ and $R_5$ represent hydrogen, a tautomer thereof and a pharmaceutically acceptable salt thereof.

**13.** A compound according to claim 11 being 2-(4-methoxyphenyl)-2,3,6,7,8,9,10,11-octahydrocycloocta[b]-pyrazolo[3,4-d]pyridin-3(5H)-one or a pharmaceutically acceptable salt thereof.

**14.** A compound according to claim 11 being 2-(4-chlorophenyl)-2,3,6,7,8,9,10,11-octahydrocycloocta[b]-pyrazolo[3,4-d]-pyridin-3(5H)-one or a pharmaceutically acceptable salt thereof.

**15.** A compound according to claim 12 being 2-pyridyl-2,3,6,7,8,9,10,11-octahydrocycloocta[b]pyrazolo[3,4-d]pyridin-3(5H)-one or a pharmaceutically acceptable salt thereof.

**16.** A pharmaceutical composition containing a compound as claimed in any one of claims 1-11, 13 and 14, in admixture or conjuction with one or more pharmaceutically suitable carriers.

**17.** A pharmaceutical composition containing a compound as claimed in any one of claims 13 and 16, in admixture or conjuction with one or more pharmaceutically suitable carriers.

**18.** Use of a compound as claimed in any one of claims 1-11, 13 and 14 for preparation of pharmaceutical compositions, suitable for enteral, parenteral or transdermal application.

**19.** Use of a compound as claimed in any one of claims 12 and 15 for preparation of pharmaceutical compositions, suitable for enteral, parenteral or transdermal application.

**20.** A compound as claimed in any one of claims 1-11, 13 and 14 for use as benzodiazepine receptor modulator.

**21.** A compound as claimed in any one of claims 12 and 15 for use as benzodiazepine receptor modulator.

**22.** A compound as claimed in any one of claims 1-11, 13 and 14 for use in a therapeutic method of

treating the human or animal body.

**23.** A compound as claimed in any one of claims 12 and 15 for use in a therapeutic method of treating the human or animal body.

**24.** analogy-process for the manufacture of a compound of the formula IA or IB claimed in claim 1, in which all the symbols have the meanings given in claim 1, pharmaceutically acceptable salts thereof, the term "lower" defines such with up to and including 7 carbon atoms, which consist in

a) reacting a compound of formula III

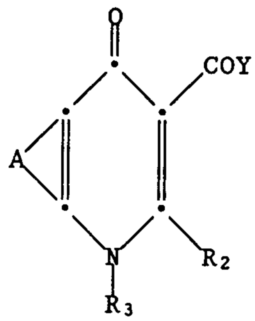

(III)

wherein A, $R_2$ and $R_3$ have meaning as previously defined, and Y is lower alkoxy; with a compound of formula IV

$R_3$'-NH-NH-$R_1$ (IV)

wherein $R_1$ has meaning as previously defined, and $R_3$' is hydrogen; or

b) reacting a compound of the formula V

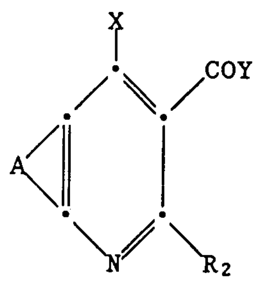

(V)

wherein A and $R_2$ have meaning as previously defined; X represents reactive etherified or esterified hydroxy; and Y represents lower alkoxy; with a compound of formula IV wherein $R_1$ has meaning as previously defined, and $R_3$' represents hydrogen or lower alkyl; or

c) cyclizing a compound of formula V above, wherein X is -$NR_3$'-$NHR_1$ and Y is lower alkoxy or hydroxy; or X is hydroxy, reactive esterified or etherified hydroxy, and Y is -$NR_1NHR_3$', and wherein A, $R_1$, $R_2$ and $R_3$' have meaning as previously defined; or

d) cyclizing a compound of formula V wherein X is lower alkoxyamino or azido and Y is -NH-$R_1$, and A, $R_1$ and $R_2$ have meaning as previously defined; or

e) cyclizing a compound of formula VI

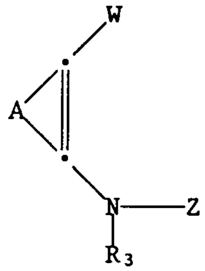

(VI)

wherein W is hydrogen, Z is

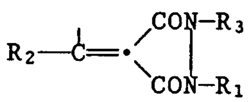

and A, $R_1$, $R_2$, $R_3$ and $R_3$' have meaning as previously defined; or
f) cyclizing a compound of formula VI above wherein W is

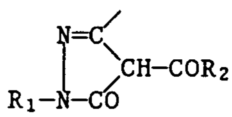

or an enamine derivative thereof, and Z is hydrogen, and A, $R_1$, $R_2$ and $R_3$ have same meaning as previously defined; or
g) cyclizing a compound of formula VI above wherein W is

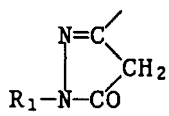

Z is $R_2CO$-, or $R_3$-N-Z is isocyano, and A, $R_1$, $R_2$ and $R_3$ have same meaning as previously defined; and if desired, converting a resulting compound of formula Ia or IB into a salt thereof or liberating a free compound from such a salt; or converting a resulting compound into another compound of the invention.

**25.** The compounds prepared according to claim 24.

**Claims for the following Contracting States : ES, GR**

**1.** Process for the manufacture of a compound of the formula

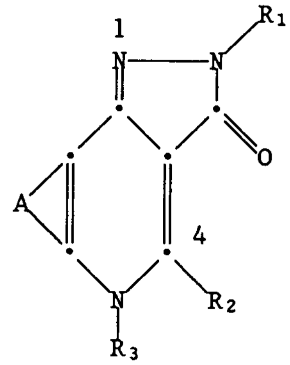 (IA) 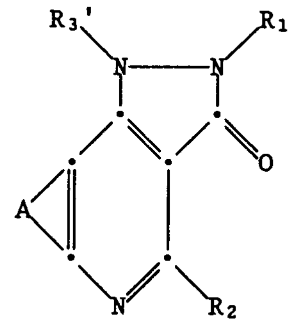 (IB)

wherein A represents an optionally substituted saturated divalent grouping which together with the two carbon atoms to which it is attached represents a fused 8, 9, 10, 11 or 12 membered carbocyclic ring selected from cycloocteno, cyclononeno, cyclodeceno, cycloundeceno and cyclododeceno; each unsubstituted or mono-, di-, tri-or tetra-substituted on carbon atoms within A by lower alkyl, lower alkylidene, $C_3$-$C_7$-cycloalkyl, hydroxy, lower alkanoyloxy, benzoyloxy unsubstituted or substituted by one or two lower alkoxy alkyl, lower alkoxy, halogen or trifluormethyl, oxo, lower alkoxy, aryl or aryl-lower alkoxy; and when disubstituted on the same carbon atom within A, said carbon atom in each ring is substituted by two lower alkyl or two aryl-lower alkyl groups, or by one lower alkyl or aryl-lower alkyl group and one group selected from hydroxy, lower alkoxy, aryl-lower alkoxy the term aryl within the above definitions represents phenyl or phenyl mono- or disubstituted by lower alkyl, lower alkoxy, hydroxy, lower alkanoyloxy, benzoyloxy unsubstituted or substituted by one or two lower alkyl, lower alkoxy, halogen or trifluoromethyl and lower alkanoyloxy, benzoyloxy unsubstituted or substituted by one or two lower alkoxy alkyl, lower alkoxy, halogen or trifluormethyl; or each disubstituted on the same carbon atom within A by straight chain alkylene of 2 to 6 carbon atoms forming with the carbon to which the alkylene chain is attached a spiro-fused 3 to 7 membered ring; or each ring is disubstituted on adjacent carbon atoms by alkylene of 3,4 or 5 carbon atoms to form with the two adjacent carbon atoms to which said alkylene grouping is attached a fused 5-, 6-, or 7-membered ring; $R_1$ represents lower alkyl, phenyl, or phenyl substituted by one or two radicals selected from lower alkyl, lower alkoxy, halogen and trifluoromethyl; or $R_1$ represents lower alkyl, phenyl, or phenyl mono- or di-substituted by lower alkyl, lower alkoxy, halogen or trifluoromethyl; or $R_1$ represents an aromatic heterocyclic radical selected from pyridyl, quinolyl, isoquinolyl, pyrimidyl and thiazolyl, or any said heterocyclic radical mono- or di-substituted on carbon by lower alkyl, lower alkoxy or halogen; and $R_2$, $R_3$ and $R_3'$ independently represent hydrogen or lower alkyl; or a pharmaceutically acceptable salt thereof, the term "lower" defines such with up to and including 7 carbon atoms, which consist in

a) reacting a compound of formula III

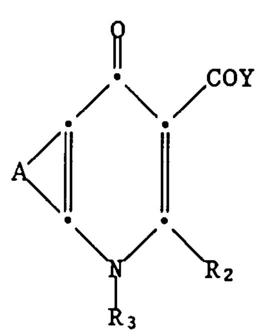 (III)

wherein A, $R_2$ and $R_3$ have meaning as previously defined, and Y is lower alkoxy; with a compound of formula IV

$R_3'$-NH-NH-$R_1$ (IV)

wherein $R_1$ has meaning as previously defined, and $R_3'$ is hydrogen; or

b) reacting a compound of the formula V

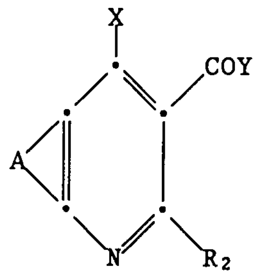

(V)

wherein A and $R_2$ have meaning as previously defined; X represents reactive etherified or esterified hydroxy; and Y represents lower alkoxy; with a compound of formula IV wherein $R_1$ has meaning as previously defined, and $R_3'$ represents hydrogen or lower alkyl; or

c) cyclizing a compound of formula V above, wherein X is $-NR_3'-NHR_1$ and Y is lower alkoxy or hydroxy; or X is hydroxy, reactive esterified or etherified hydroxy, and Y is $-NR_1NHR_3'$, and wherein A, $R_1$, $R_2$ and $R_3'$ have meaning as previously defined; or

d) cyclizing a compound of formula V wherein X is lower alkoxyamino or azido and Y is $-NH-R_1$, and A, $R_1$ and $R_2$ have meaning as previously defined; or

e) cyclizing a compound of formula VI

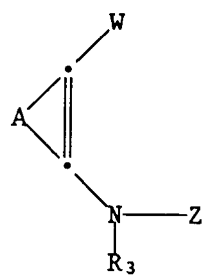

(VI)

wherein W is hydrogen, Z is

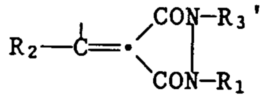

and A, $R_1$, $R_2$, $R_3$ and $R_3'$ have meaning as previously defined; or

f) cyclizing a compound of formula VI above wherein W is

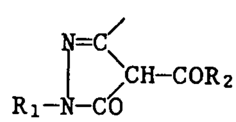

or an enamine derivative thereof, and Z is hydrogen, and A, $R_1$, $R_2$ and $R_3$ have same meaning as previously defined; or

g) cyclizing a compound of formula VI above wherein W is

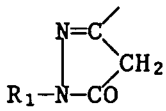

Z is $R_2CO$-, or $R_3$-N-Z is isocyano, and A, $R_1$, $R_2$ and $R_3$ have same meaning as previously defined; and if desired, converting a resulting compound of formula Ia or IB into a salt thereof or liberating a free compound from such a salt; or converting a resulting compound into another compound of the invention.

**2.** Process according to claim 1, wherein there is prepared a compound of formula IA or IB wherein A together with the two carbon atoms to which it is attached represents a fused ring selected from cycloocteno, cyclononeno, cyclodeceno, cycloundeceno and cyclododeceno in which A represents hexamethylene, heptamethylene, octamethylene, nonamethylene or decamethylene respectively; each said ring unsubstituted or mono-, di-, tri- or tetra-substituted on carbon atoms within A by lower alkyl, lower alkylidene, $C_3$-$C_7$-cycloalkyl, hydroxy, lower alkanoyloxy, benzoyloxy unsubstituted or substituted by one or two lower alkoxy alkyl, lower alkoxy, halogen or trifluormethyl, oxo, lower alkoxy, aryl, aryl-lower alkyl or aryl-lower alkoxy; and when disubstituted on the same carbon atom within A, said carbon atom in each ring is preferably substituted by two lower alkyl or two aryl-lower alkyl groups, or by one lower alkyl or aryl-lower alkyl and one group selected from hydroxy, lower alkoxy, aryl-lower alkoxy the term aryl within the above definitions represents phenyl or phenyl mono- or disubstituted by lower alkyl, lower alkoxy, hydroxy, lower alkanoyloxy, benzoyloxy unsubstituted or substituted by one or two lower alkyl, lower alkoxy, halogen or trifluoromethyl and lower alkanoyloxy, benzoyloxy unsubstituted or substituted by one or two lower alkoxy alkyl, lower alkoxy, halogen or trifluormethyl or each ring is disubstituted on the same carbon atom within A by ethylene, propylene, butylene or pentylene forming with the carbon to which the alkylene chain is attached a spiro fused cyclopropyl, cyclobutyl, cyclopentyl or cyclohexyl ring; or each ring is disubstituted on adjacent carbon atoms by propylene or butylene to form with the two adjacent carbon atoms to which said alkylene grouping is attached a fused cyclopentane or cyclohexane ring; $R_1$, $R_2$, $R_3$ and $R_3$' have meaning as defined in claim 1; or a pharmaceutically acceptable salt thereof, the term "lower" defines such with up to and including 7 carbon atoms.

**3.** Process according to claim 1, wherein there is prepared a compound of the formula II

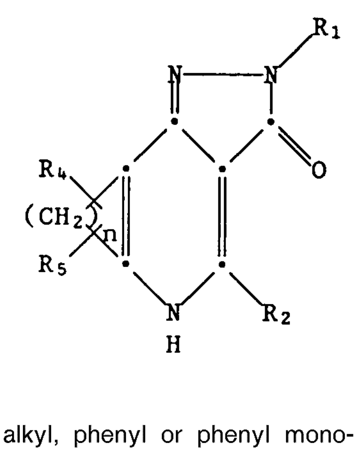

(II)

wherein $R_1$ represents lower alkyl, phenyl or phenyl mono- or di-substituted by lower alkyl, lower alkoxy, halogen or trifluoromethyl; or $R_1$ represents an aromatic heterocyclic radical selected from pyridyl, quinolyl, isoquinolyl, pyrimidyl and thiazolyl, or any said radical mono- or di-substituted by lower alkyl, lower alkoxy or halogen; $R_4$ and $R_5$ represent independently hydrogen, lower alkyl, $C_3$-$C_7$-cycloalkyl, hydroxy, lower alkanoyloxy, benzoyloxy unsubstituted or substituted by one or two lower alkoxy alkyl, lower alkoxy, halogen or trifluormethyl lower alkoxy, aryl, aryl-lower alkyl or aryl-lower alkoxy the term aryl within the above definitions represents phenyl or phenyl mono- or disubstituted by lower alkyl, lower alkoxy, hydroxy, lower alkanoyloxy, benzoyloxy unsubstituted or substituted by one or two lower alkyl, lower alkoxy, halogen or trifluoromethyl or $R_4$ and $R_5$ when combined and attached to the same carbon atom represent spiro-fused cyclopentyl or spiro-fused cyclohexyl; n represents the

integer 6, 7, 8, 9 and 10; aryl represents phenyl or phenyl mono- or di-substituted by lower alkyl, lower alkoxy, hydroxy, halogen or trifluoromethyl; tautomers thereof; or a pharmaceutically acceptable salt thereof, the term "lower" defines such with up to and including 7 carbon atoms.

**4.** Process according to claim 3, wherein there is prepared a compound of formula II wherein n represents the integer 6.

**5.** Process according to claim 3, wherein there is prepared a compound of formula II wherein n represents the integer 7.

**6.** Process according to claim 3, wherein there is prepared a compound of formula II wherein n represents the integer 8.

**7.** Process according to claim 3, wherein there is prepared a compound of formula II wherein n represents the integer 9.

**8.** Process according to claim 3, wherein there is prepared a compound of formula II wherein n represents the integer 10.

**9.** Process according to claim 3, wherein there is prepared a compound of formula II wherein n represents an integer as defined therein; $R_1$ represents phenyl, or phenyl mono- or di-substituted by lower alkyl, lower alkoxy, halogen or trifluormethyl; $R_4$ and $R_5$ represent hydrogen or lower alkyl; a tautomer thereof; or a pharmaceutically acceptable salt thereof, the term "lower" defines such with up to and including 7 carbon atoms.

**10.** Process according to claim 3, wherein there is prepared a compound of formula II wherein n represents an integer as defined therein; $R_1$ represents phenyl or phenyl monosubstituted by lower alkyl, lower alkoxy or halogen; $R_4$ and $R_5$ represent hydrogen or lower alkyl; a tautomer thereof; or a pharmaceutically acceptable salt thereof, the term "lower" defines such with up to and including 7 carbon atoms.

**11.** Process according to claim 10, wherein there is prepared a compound of formula II wherein $R_1$ represents phenyl mono-substituted at the para position by lower alkyl, lower alkoxy or halogen; and $R_4$ and $R_5$ represent hydrogen, a tautomer therof, or a pharmaceutically acceptable salt therof, the term "lower" defines such with up to and including 7 carbon atoms.

**12.** Process according to claim 3, wherein there is prepared a compound of formula II wherein n represent an integer as defined therein, $R_1$ represents 2-pyridyl, $R_4$ and $R_5$ represent hydrogen, a tautomer thereof and a pharmaceutically acceptable salt thereof.

**13.** Process according to claim 1 wherein 2-(4-methoxyphenyl)-2,3,6,7,8,9,10,11-octahydrocycloocta[b]-pyrazolo[3,4-d]pyridin-3(5H)-one or a pharmaceutically acceptable salt thereof is prepared.

**14.** Process according to claim 1 wherein 2-(4-chlorophenyl)-2,3,6,7,8,9,10,11-octahydrocycloocta[b]-pyrazolo[3,4-d]-pyridin-3(5H)-one or a pharmaceutically acceptable salt thereof is prepared.

**15.** Process according to claim 1 wherein 2-pyridyl-2,3,6,7,8,9,10,11-octahydrocycloocta[b]pyrazolo[3,4-d]-pyridin-3(5H)-one or a pharmaceutically acceptable salt thereof is prepared.

**16.** Process for manufacture of a pharmaceutical composition containing a compound as prepared according to any one of claims 1-11, 13 and 14, in admixture or conjuction with one or more pharmaceutically suitable carriers.

**17.** Process for manufacture of a pharmaceutical composition containing a compound as prepared according to any one of claims 12 and 15, in admixture or conjuction with one or more pharmaceutically suitable carriers.

**Patentansprüche**

**Patentansprüche für folgende Vertragsstaaten : AT, BE, CH, DE, FR, GB, IT, LI, LU, NL, SE**

**1.** Verbindung der Formel

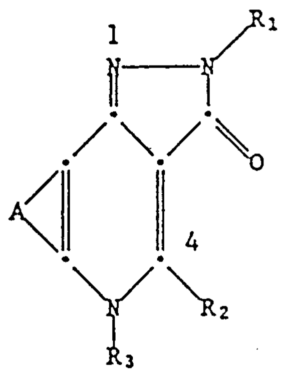

(IA)

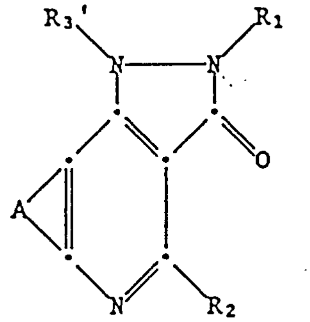

(IB)

worin A eine gegebenenfalls substituierte, gesättigte, divalente Gruppierung darstellt, die zusammen mit den zwei Kohlenstoffatomen, an die sie gebunden ist, einen kondensierten 8-, 9-, 10-, 11- oder 12gliedrigen carbocyclischen Ring darstellt, der ausgewählt wird aus Cycloocteno, Cyclononeno, Cyclodeceno, Cycloundeceno und Cyclododeceno; jeder unsubstituiert oder an Kohlenstoffatomen innerhalb von A mono-, di-, tri- oder tetrasubstituiert durch niedere Alkyl-, niedere Alkyliden-, $C_3$-$C_7$-Cycloalkyl-, Hydroxy-, niedere Alkanoyloxy-Gruppen, Benzoyloxy-Gruppen, die unsubstituiert sind oder substituiert durch eine oder zwei niedere Alkoxyalkyl-, niedere Alkoxy-, Halogen- oder Trifluormethyl-Gruppen, Oxo-, niedere Alkoxy-, Aryl- oder Aryl-niederalkoxy-Gruppen; und wenn am gleichen Kohlenstoffatom innerhalb von A disubstituiert, ist besagtes Kohlenstoffatom in jedem Ring substituiert durch zwei niedere Alkyl- oder zwei Aryl-niederalkyl-Gruppen, oder durch eine niedere Alkyl- oder Aryl-niederalkyl-Gruppe und eine Gruppe, die ausgewählt wird aus Hydroxy, niederem Alkoxy, Aryl-niederalkoxy, wobei der Ausdruck Aryl innerhalb der obigen Definitionen Phenyl darstellt oder Phenyl, das mono- oder disubstituiert ist durch niedere Alkyl-, niedere Alkoxy-, Hydroxy-, niedere Alkanoyloxy-Gruppen, Benzoyloxy-Gruppen, die unsubstituiert sind oder substituiert durch eine oder zwei niedere Alkyl-, niedere Alkoxy-, Halogen- oder Trifluormethyl- und niedere Alkanoyloxy-Gruppen, Benzoyloxy, das unsubstituiertist oder substituiert durch eine oder zwei niedere Alkoxy-alkyl-, niedere Alkoxy-, Halogen- oder Trifluormethyl-Gruppen, oder jeweils am gleichen Kohlenstoffatom innerhalb von A disubstituiert durch geradkettiges Alkylen von 2 bis 6 Kohlenstoffatomen, mit dem Kohlenstoff, an das die Alkylenkette gebunden ist, einen spirokondensierten 3- bis 7gliedrigen Ring bildend; oder jeder Ring an benachbarten Kohlenstoffatomen durch Alkylen mit 3, 4 oder 5 Kohlenstoffatomen disubstituiert ist, um mit den zwei benachbarten Kohlenstoffatomen, an die die Alkylengruppierung gebunden ist, einen kondensierten 5-, 6- oder 7gliedrigen Ring zu bilden; $R_1$ niederes Alkyl, Phenyl oder Phenyl, das mono- oder disubstituiert ist durch niederes Alkyl, niederes Alkoxy, Halogen oder Trifluormethyl, darstellt; oder $R_1$ einen aromatischen heterocyclischen Rest darstellt, der ausgewählt wird aus Pyridyl, Chinolyl, Isochinolyl, Pyrimidyl und Thiazolyl, oder irgendeinen genannten heterocyclischen Rest, der an Kohlenstoff mono- oder disubstituiert ist durch niederes Alkyl, niederes Alkoxy oder Halogen; und $R_2$, $R_3$ und $R_3'$ unabhängig Wasserstoff oder niederes Alkyl darstellen; oder ein pharmazeutisch annehmbares Salz davon, wobei der Ausdruck "niederes" solche mit bis zu und inklusive 7 Kohlenstoffatomen definiert.

**2.** Verbindung nach Anspruch 1 mit der Formel IA oder IB, worin A zusammen mit den zwei Kohlenstoffatomen, an die es gebunden ist, einen kondensierten Ring darstellt, der aus gewählt wird aus Cycloocteno, Cyclononeno, Cyclodeceno, Cycloundeceno und Cyclododeceno, worin A Hexamethylen, Heptamethylen, Octamethylen, Nonamethylen bzw. Decamethylen darstellt; jeder genannte Ring unsubstituiert ist oder an Kohlenstoffatomen innerhalb von A mono-, di-, tri- oder tetrasubstituiert ist durch niedere Alkyl-, niedere Alkyliden-, $C_3$-$C_7$-Cycloalkyl, Hydroxy-, niedere Alkanoyloxy-Gruppen, Benzoyloxy-Gruppen, die unsubstituiert sind oder substituiert durch eine oder zwei niedere Alkoxy-alkyl-, niedere Alkoxy, Halogen- oder Trifluormethyl-Gruppen, Oxo-, niedere Alkoxy-, Aryl-, Aryl-niederalkyl- oder Aryl-niederalkoxy-Gruppen; und wenn am selben Kohlenstoffatom innerhalb von A disubstituiert, ist besagtes Kohlenstoffatom in jedem Ring vorzugsweise substituiert durch zwei niedere Alkyl- oder zwei Aryl-niederalkyl-Gruppen, oder durch eine niedere Alkyl- oder Aryl-niederalkyl-Gruppe und eine Gruppe, die ausgewählt wird aus Hydroxy, niederem Alkoxy, Aryl-niederalkoxy, wobei der

Ausdruck Aryl innerhalb der obigen Definitionen Phenyl darstellt oder Phenyl, das mono- oder disubstituiert ist durch niedere Alkyl-, niedere Alkoxy-, Hydroxy-, niedere Alkanoyloxy-Gruppen, Benzoyloxy-Gruppen, die unsubstituiert sind oder substituiert durch eine oder zwei niedere Alkyl-, niedere Alkoxy-, Halogen- oder Trifluormethyl-Gruppen und niedere Alkanoyloxy-Gruppen, Benzoyloxy, das unsubstituiert ist oder substituiert durch eine oder zwei niedere Alkoxy-alkyl-, niedere Alkoxy-, Halogen- oder Trifluormethyl-Gruppen; oder jeder Ring am selben Kohlenstoffatom innerhalb von A disubstituiert ist durch Ethylen, Propylen, Butylen oder Pentylen, mit dem Kohlenstoff, an den die Alkylenkette gebunden ist, einen spiro-kondensierten Cyclopropyl-, Cyclobutyl-, Cyclopentyl- oder Cyclohexylring bildend; oder jeder Ring an benachbarten Kohlenstoffatomen disubstituiert ist durch Propylen oder Butylen, um mit den zwei benachbarten Kohlenstoffatomen, an die die Alkylengruppierung gebunden ist, einen kondensierten Cyclopentan- oder Cyclohexanring zu bilden; $R_1$, $R_2$, $R_3$ und $R_3$' die im Anspruch 1 definierte Bedeutung haben; oder ein pharmazeutisch annehmbares Salz davon, wobei der Ausdruck "niederes" solche mit bis zu und inklusive 7 Kohlenstoffatomen definiert.

3. Verbindung nach Anspruch 1 mit der Formel

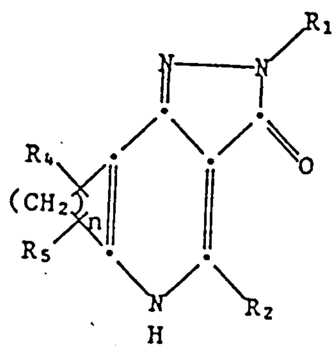

(II)

worin $R_1$ niederes Alkyl, Phenyl oder Phenyl, das mono- oder disubstituiert ist durch niederes Alkyl, niederes Alkoxy, Halogen oder Trifluormethyl, darstellt; oder $R_1$ einen aromatischen heterocyclischen Rest darstellt, der ausgewählt wird aus Pyridyl, Chinolyl, Isochinolyl, Pyrimidyl und Thiazolyl, oder irgendeinen genannten Rest, der mono- oder disubstituiert ist durch niederes Alkyl, niederes Alkoxy oder Halogen; $R_4$ und $R_5$ unabhängig Wasserstoff, niederes Alkyl, $C_3$-$C_7$-Cycloalkyl, Hydroxy, niederes Alkanoyloxy, Benzoyloxy, das unsubstituiert ist oder substituiert durch eine oder zwei niedere Alkoxy-alkyl-, niedere Alkoxy-, Halogen- oder Trifluormethyl-Gruppen, niederes Alkoxy, Aryl-, Aryl-niederalkyl oder Aryl-niederalkoxy darstellen, wobei der Ausdruck Aryl innerhalb der obigen Definitionen Phenyl darstellt oder Phenyl, das mono- oder disubstituiert ist durch niederes Alkyl, niederes Alkoxy, Hydroxy, niederes Alkanoyloxy, Benzoyloxy, das unsubstituiert ist oder substituiert durch eine oder zwei niedere Alkyl-, niedere Alkoxy-, Halogen oder Trifluormethyl-Gruppen; oder $R_4$ und $R_5$, wenn sie vereinigt und an dasselbe Kohlenstoffatom gebunden sind, spiro-kondensiertes Cyclopentyl oder spiro-kondensiertes Cyclohexyl darstellen; n die ganze Zahl 6, 7, 8, 9 und 10 darstellt; Aryl Phenyl darstellt oder Phenyl, das mono- oder disubstituiert ist durch niederes Alkyl, niederes Alkoxy, Hydroxy, Halogen oder Trifluormethyl; Tautomere davon; oder ein pharmazeutisch annehmbares Salz davon; wobei der Ausdruck "niederes" solche mit bis zu und inklusive 7 Kohlenstoffatomen definiert.

4. Verbindung nach Anspruch 3, worin n die ganze Zahl 6 darstellt.

5. Verbindung nach Anspruch 3, worin n die ganze Zahl 7 darstellt.

6. Verbindung nach Anspruch 3, worin n die ganze Zahl 8 darstellt.

7. Verbindung nach Anspruch 3, worin n die ganze Zahl 9 darstellt.

8. Verbindung nach Anspruch 3, worin n die ganze Zahl 10 darstellt.

9. Verbindung nach Anspruch 3, worin n eine ganze Zahl wie hier definiert darstellt; $R_1$ Phenyl darstellt oder Phenyl, das mono- oder disubstituiert ist durch niederes Alkyl, niederes Alkoxy, Halogen oder

Trifluormethyl; $R_4$ und $R_5$ Wasserstoff oder niederes Alkyl darstellen; ein Tautomer davon; oder ein pharmazeutisch annehmbares Salz davon, wobei der Ausdruck "niederes" solche mit bis zu und inklusive 7 Kohlenstoffatomen definiert.

**10.** Verbindung nach Anspruch 3, worin n eine ganze Zahl wie hier definiert darstellt; $R_1$ Phenyl darstellt oder Phenyl, das monosubstituiert ist durch niederes Alkyl, niederes Alkoxy oder Halogen; $R_4$ und $R_5$ Wasserstoff oder niederes Alkyl darstellen; ein Tautomer davon; oder ein pharmazeutisch annehmbares Salz davon, wobei der Ausdruck "niederes" solche mit bis zu und inklusive 7 Kohlenstoffatomen definiert.

**11.** Verbindung nach Anspruch 10, worin $R_1$ Phenyl, das in der para-Position monosubstituiert ist durch niederes Alkyl, niederes Alkoxy oder Halogen, darstellt; und $R_4$ und $R_5$ Wasserstoff darstellen; ein Tautomer davon, oder ein pharmazeutisch annehmbares Salz davon, wobei der Ausdruck "niederes" solche mit bis zu und inklusive 7 Kohlenstoffatomen definiert.

**12.** Verbindung nach Anspruch 3, worin n eine ganze Zahl wie hier definiert darstellt, $R_1$ 2-Pyridyl darstellt, $R_4$ und $R_5$ Wasserstoff darstellen, ein Tautomer davon und ein pharmazeutisch annehmbares Salz davon.

**13.** Verbindung nach Anspruch 11, die 2-(4-Methoxyphenyl)-2,3,6,7,8,9,10,11-octahydrocycloocta[b]-pyrazolo[3,4-d]pyridin-3(5H)-on oder ein pharmazeutisch annehmbares Salz davon ist.

**14.** Verbindung nach Anspruch 11, die 2-(4-Chlorphenyl)-2,3,6,7,8,9,10,11-octahydrocycloocta[b]pyrazolo-[3,4-d]pyridin-3(5H)-on oder ein pharmazeutisch annehmbares Salz davon ist.

**15.** Verbindung nach Anspruch 12, die 2-Pyridyl-2,3,6,7,8,9,10,11-octahydrocycloocta[b]pyrazolo[3,4-d]-pyridin-3(5H)-on oder ein pharmazeutisch annehmbares Salz davon ist.

**16.** Pharmazeutische Zusammensetzung, die eine Verbindung nach irgendeinem der Ansprüche 1-11, 13 und 14 beigemischt zu oder in Verbindung mit einem oder mehreren pharmazeutisch geeigneten Trägem enthält.

**17.** Pharmazeutische Zusammensetzung, die eine Verbindung nach irgendeinem der Ansprüche 13 und 16 beigemischt zu oder in Verbindung mit einem oder mehreren pharmazeutisch geeigneten Trägern enthält.

**18.** Verwendung einer Verbindung nach irgendeinem der Ansprüche 1-11, 13 und 14 zur Herstellung pharmazeutischer Zusammensetzungen, die sich für enterale, parenterale oder transdermale Verabreichung eignen.

**19.** Verwendung einer Verbindung nach irgendeinem der Ansprüche 12 und 15 zur Herstellung pharmazeutischer Zusammensetzungen, die sich für enterale, parenterale oder transdermale Verabreichung eignen.

**20.** Verbindung nach irgendeinem der Ansprüche 1-11, 13 und 14 zur Verwendung als Benzodiaze pin-Rezeptor-Modulator.

**21.** Verbindung nach irgendeinem der Ansprüche 12 und 15 zur Verwendung als Benzodiazepin-Rezeptor-Modulator.

**22.** Verbindung nach irgendeinem der Ansprüche 1-11, 13 und 14 zur Verwendung bei einer therapeutischen Methode zur Behandlung des menschlichen oder tierischen Körpers.

**23.** Verbindung nach irgendeinem der Ansprüche 12 und 15 zur Verwendung bei einer therapeutischen Methode zur Behandlung des menschlichen oder tierischen Körpers.

**24.** Analogieverfahren zur Herstellung einer Verbindung der Formel IA oder IB, die in Anspruch 1 beansprucht wird, in der alle Symbole die in Anspruch 1 gegebene Bedeutung haben, pharmazeutisch

annehmbarer Salze davon, wobei der Ausdruck "niedere" solche mit bis zu und inklusive 7 Kohlenstoffatomen definiert, welches Verfahren umfaßt: a) Umsetzung einer Verbindung der Formel III

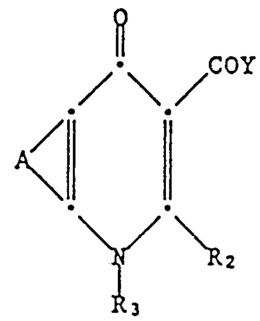

(III)

worin A, $R_2$ und $R_3$ die zuvor definierte Bedeutung haben und Y niederes Alkoxy ist, mit einer Verbindung der Formel IV

$R_3$'-NH-NH-$R_1$ (IV)

worin $R_1$ die zuvor definierte Bedeutung hat und $R_3$' Wasserstoff ist; oder b) Umsetzung einer Verbindung der Formel V

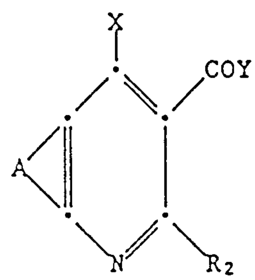

(V)

worin A und $R_2$ zuvor definierte Bedeutung haben; X reaktives verethertes oder verestertes Hydroxy darstellt; und Y niederes Alkoxy darstellt; mit einer Verbindung der Formel IV, in der $R_1$ zuvor definierte Bedeutung hat und $R_3$' Wasserstoff oder niederes Alkyl darstellt; oder c) Cyclisieren einer Verbindung der obigen Formel V, in der X für -N$R_3$'-NH$R_1$ steht und Y niederes Alkoxy oder Hydroxy ist; oder X Hydroxy, reaktives verestertes oder verethertes Hydroxy und Y -N$R_1$NH$R_3$' ist und worin A, $R_1$, $R_2$ und $R_3$' zuvor definierte Bedeutung haben; oder d) Cyclisieren einer Verbindung der Formel V, in der X niederes Alkoxyamino oder Azido ist und Y für -NH-$R_1$ steht und A, $R_1$ und $R_2$ zuvor definierte Bedeutung haben; oder e) Cyclisieren einer Verbindung der Formel VI

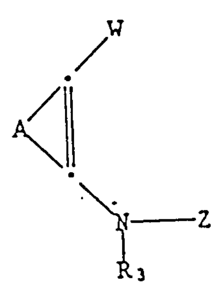

(VI)

worin W Wasserstoff ist, Z für

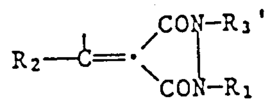

steht und A, $R_1$, $R_2$, $R_3$ und $R_3$' zuvor definierte Bedeutung haben; oder f) Cyclisieren einer Verbindung der Formel VI, in der W

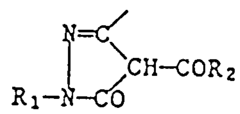

ist, oder ein Enamin-Derivat davon, und Z Wasserstoff ist und A, $R_1$, $R_2$ und $R_3$ die gleiche Bedeutung wie zuvor definiert haben; oder g) Cyclisieren einer Verbindung der Formel VI oben, in der W

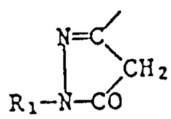

ist, Z $R_2$CO- ist oder $R_3$-N-Z Isocyano ist und A, $R_1$, $R_2$ und $R_3$ zuvor definierte Bedeutung haben; und, falls das gewünscht wird, Umwandlung einer resultierenden Verbindung der Formel IA oder IB in ein Salz davon oder Freisetzung einer freien Verbindung aus einem solchen Salz; oder Umwandlung einer resultierenden Verbindung in eine andere erfindungsgemäße Verbindung.

**25.** Verbindungen, die nach Anspruch 24 hergestellt werden.

**Patentansprüche für folgende Vertragsstaaten : ES, GR**

**1.** Verfahren zur Herstellung einer Verbindung der Formel

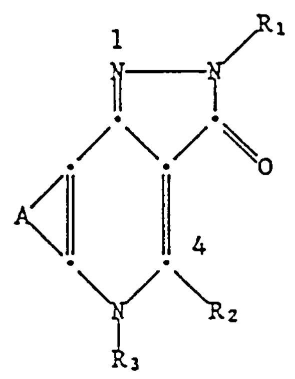

(IA)

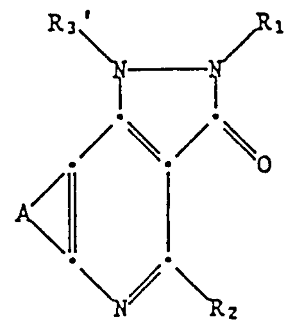

(IB)

worin A eine gegebenenfalls substituierte, gesättigte, divalente Gruppierung darstellt, die zusammen mitden zwei Kohlenstoffatomen, an die sie gebunden ist, einen kondensierten 8-, 9-, 10-, 11- oder 12gliedrigen carbocyclischen Ring darstellt, der ausgewählt wird aus Cycloocteno, Cyclononeno, Cyclodeceno, Cycloundeceno und Cyclododeceno; jeder unsubstituiert oder an Kohlenstoffatomen innerhalb von A mono-, di-, tri- oder tetrasubstituiert durch niedere Alkyl-, niedere Alkyliden-, $C_3$-$C_7$-Cycloalkyl, Hydroxy-, niedere Alkanoyloxy-Gruppen, Benzoyloxy-Gruppen, die unsubstituiert sind oder substituiert durch eine oder zwei niedere Alkoxyalkyl-, niedere Alkoxy-, Halogen- oder Trifluormethyl-Gruppen, Oxo-, niedere Alkoxy-, Aryl- oder Aryl-niederalkoxy-Gruppen; und wenn am gleichen Kohlenstoffatom innerhalb von A disubstituiert, ist besagtes Kohlenstoffatom in jedem Ring substituiert durch

zwei niedere Alkyl- oder zwei Aryl-niederalkyl-Gruppen, oder durch eine niedere Alkyl- oder Aryl-niederalkyl-Gruppe und eine Gruppe, die ausgewählt wird aus Hydroxy, niederem Alkoxy, Aryl-niederalkoxy, wobei der Ausdruck Aryl innerhalb der obigen Definitionen Phenyl darstellt oder Phenyl, das mono- oder disubstituiert ist durch niedere Alkyl-, niedere Alkoxy-, Hydroxy-, niedere Alkanoyloxy-Gruppen, Benzoyloxy-Gruppen, die unsubstituiert sind oder substituiert durch eine oder zwei niedere Alkyl-, niedere Alkoxy-, Halogen- oder Trifluormethyl- und niedere Alkanoyloxy-Gruppen, Benzoyloxy, das unsubstituiert ist oder substituiert durch eine oder zwei niedere Alkoxy-alkyl-, niedere Alkoxy-, Halogen- oder Trifluormethyl-Gruppen, oder jeweils am gleichen Kohlenstoffatom innerhalb von A disubstituiert durch geradkettiges Alkylen von 2 bis 6 Kohlenstoffatomen, mit dem Kohlenstoff, an das die Alkylenkette gebunden ist, einen spiro-kondensierten 3- bis 7gliedrigen Ring bildend; oder jeder Ring an benachbarten Kohlenstoffatomen durch Alkylen mit 3, 4 oder 5 Kohlenstoffatomen disubstituiert ist, um mit den zwei benachbarten Kohlenstoffatomen, an die die Alkylengruppierung gebunden ist, einen kondensierten 5-, 6- oder 7gliedrigen Ring zu bilden; $R_1$ niederes Alkyl, Phenyl oder Phenyl, das substituiert ist durch einen oder zwei Reste, die ausgewählt werden aus niederem Alkyl, niederem Alkoxy, Halogen oder Trifluormethyl, darstellt; oder $R_1$ niederes Alkyl, Phenyl oder Phenyl, das mono- oder disubstituiert ist durch niederes Alkyl, niederes Alkoxy, Halogen oder Trifluormethyl, darstellt; oder $R_1$ einen aromatischen heterocyclischen Rest darstellt, der ausgewählt wird aus Pyridyl, Chinolyl, Isochinolyl, Pyrimidyl und Thiazolyl, oder irgendeinen genannten heterocyclischen Rest, der an Kohlenstoff mono- oder disubstituiert ist durch niederes Alkyl, niederes Alkoxy oder Halogen; und $R_2$, $R_3$ und $R_3$' unabhängig Wasserstoff oder niederes Alkyl darstellen; oder eines pharmazeutisch annehmbaren Salzes davon, wobei der Ausdruck "niederes" solche mit bis zu und inklusive 7 Kohlenstoffatomen definiert, welches Verfahren umfaßt: a) Umsetzung einer Verbindung der Formel III

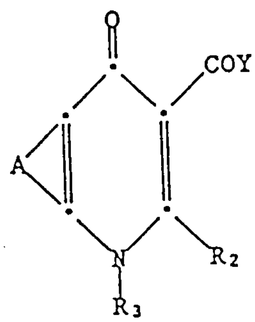

(III)

worin A, $R_2$ und $R_3$ die zuvor definierte Bedeutung haben und Y niederes Alkoxy ist, mit einer Verbindung der Formel IV

$R_3$'-NH-NH-$R_1$ (IV)

worin $R_1$ die zuvor definierte Bedeutung hat und $R_3$' Wasserstoff ist; oder b) Umsetzung einer Verbindung der Formel V

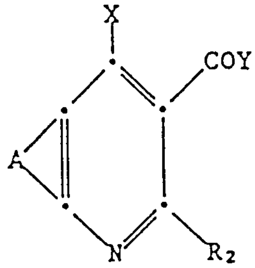

(V)

worin A und $R_2$ zuvor definierte Bedeutung haben; X reaktives verethertes oder verestertes Hydroxy darstellt; und Y niederes Alkoxy darstellt; mit einer Verbindung der Formel IV, in der $R_1$ zuvor definierte Bedeutung hat und $R_3$' Wasserstoff oder niederes Alkyl darstellt; oder c) Cyclisieren einer Verbindung der obigen Formel V, in der X für -$NR_3$'-$NHR_1$ steht und Y niederes Alkoxy oder Hydroxy ist; oder X

Hydroxy, reaktives verestertes oder verethertes Hydroxy und Y $-NR_1NHR_3'$ ist und worin A, $R_1$, $R_2$ und $R_3'$ zuvor definierte Bedeutung haben; oder d) Cyclisieren einer Verbindung der Formel V, in der X niederes Alkoxyamino oder Azido ist und Y für $-NH-R_1$ steht und A, $R_1$ und $R_2$ zuvordefinierte Bedeutung haben; oder e) Cyclisieren einer Verbindung der Formel VI

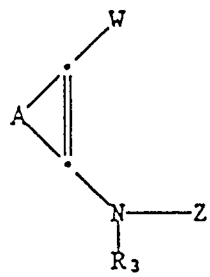

(VI)

worin W Wasserstoff ist, Z für

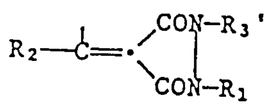

steht und A, $R_1$, $R_2$, $R_3$ und $R_3'$ zuvor definierte Bedeutung haben; oder f) Cyclisieren einer Verbindung der Formel VI, in der W

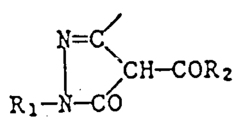

ist, oder ein Enamin-Derivat davon, und Z Wasserstoff ist und A, $R_1$, $R_2$ und $R_3$ die gleiche Bedeutung wie zuvor definiert haben; oder g) Cyclisieren einer Verbindung der Formel VI oben, in der W

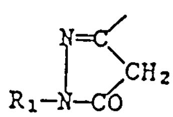

ist, Z $R_2CO$- ist oder $R_3$-N-Z Isocyano ist und A, $R_1$, $R_2$ und $R_3$ zuvor definierte Bedeutung haben; und, falls das gewünscht wird, Umwandlung einer resultierenden Verbindung der Formel IA oder IB in ein Salz davon oder Freisetzung einer freien Verbindung aus einem solchen Salz; oder Umwandlung einer resultierenden Verbindung in eine andere erfindungsgemäße Verbindung.

**2.** Verfahren nach Anspruch 1, worin eine Verbindung der Formel IA oder IB hergestellt wird, worin A zusammen mit den zwei Kohlenstoffatomen, an die es gebunden ist, einen kondensierten Ring darstellt, der ausgewählt wird aus Cycloocteno, Cyclononeno, Cyclodeceno, Cycloundeceno und Cyclododeceno, worin A Hexamethylen, Heptamethylen, Octamethylen, Nonamethylen bzw. Decamethylen darstellt; jeder genannte Ring unsubstituiert oder an Kohlenstoffatomen innerhalb von A mono-, di-, tri- oder tetrasubstituiert ist durch niedere Alkyl-, niedere Alkyliden-, $C_3$-$C_7$-Cycloalkyl-, Hydroxy-, niedere Alkanoyloxy-Gruppen, Benzoyloxy-Gruppen, die unsubstituiert sind oder substituiert durch eine oder zwei niedere Alkoxy-alkyl-, niedere Alkoxy-, Halogen- oder Trifluormethyl-Gruppen, Oxo-, niedere Alkoxy-, Aryl-, Aryl-niederalkyl- oder Aryl-niederalkoxy-Gruppen; und wenn am selben Kohlenstoffatom innerhalb von A disubstituiert, ist besagtes Kohlenstoffatom in jedem Ring vorzugsweise substituiert durch zwei niedere Alkyl- oder zwei Aryl-niederalkyl-Gruppen, oder durch eine niedere Alkyl- oder Aryl-niederalkyl-Gruppe und eine Gruppe, die ausgewählt wird aus Hydroxy, niederem Alkoxy, Aryl-niederalkoxy, wobei der Ausdruck Aryl innerhalb der obigen Definitionen Phenyl darstellt oder Phenyl, das mono- oder disubstituiert ist durch niedere Alkyl-, niedere Alkoxy-, Hydroxy-, niedere Alkanoyloxy-Gruppen, Benzoyloxy-Gruppen, die unsubstituiert sind oder substituiert durch eine oder zwei niedere Alkyl-, niedere Alkoxy-, Halogen- oder Trifluormethyl-Gruppen und niedere Alkanoyloxy-Gruppen, Benzoyloxy, das unsubstituiert ist oder substituiert durch eine oder zwei niedere Alkoxy-alkyl-, niedere Alkoxy-, Halogen oder Trifluormethyl-Gruppen; oder jeder Ring am selben Kohlenstoffatom innerhalb von A disubstituiert ist durch Ethylen, Propylen, Butylen oder Pentylen, mit dem Kohlenstoff, an den die Alkylenkette gebunden ist, einen spiro-kondensierten Cyclopropyl-, Cyclobutyl-, Cyclopentyl- oder Cyclohexylring bildend; oder jeder Ring an benachbarten Kohlenstoffatomen disubstituiert ist durch Propylen oder Butylen, um mit den zwei benachbarten Kohlenstoffatomen, an die die Alkylengruppierung gebunden ist, einen kondensierten Cyclopentan- oder Cyclohexanring zu bilden; $R_1$, $R_2$, $R_3$ und $R_3'$ die im Anspruch 1 definierte Bedeutung haben; oder ein pharmazeutisch annehmbares Salz davon, wobei der Ausdruck "niederes" solche mit bis zu und inklusive 7 Kohlenstoffatomen definiert.

3. Verfahren nach Anspruch 1, worin eine Verbindung der Formel II hergestellt wird,

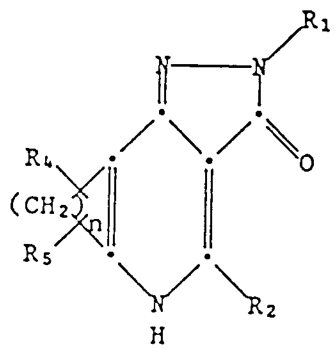

(II)

worin $R_1$ niederes Alkyl, Phenyl oder Phenyl, das mono- oder disubstituiert ist durch niederes Alkyl, niederes Alkoxy, Halogen oder Trifluormethyl, darstellt; oder $R_1$ einen aromatischen heterocyclischen Rest darstellt, der ausgewählt wird aus Pyridyl, Chinolyl, Isochinolyl, Pyrimidyl und Thiazolyl, oder irgendeinen genannten Rest, der mono- oder disubstituiert ist durch niederes Alkyl, niederes Alkoxy oder Halogen; $R_4$ und $R_5$ unabhängig Wasserstoff, niederes Alkyl, $C_3$-$C_7$-Cycloalkyl, Hydroxy, niederes Alkanoyloxy, Benzoyloxy, das unsubstituiert ist oder substituiert durch eine oder zwei niedere Alkoxy-alkyl-, niedere Alkoxy-, Halogen- oder Trifluormethyl-Gruppen, niederes Alkoxy, Aryl-, Aryl-niederalkyl oder Aryl-niederalkoxy darstellen, wobei der Ausdruck Aryl innerhalb der obigen Definitionen Phenyl darstellt oder Phenyl, das mono- oder disubstituiert ist durch niederes Alkyl, niederes Alkoxy, Hydroxy, niederes Alkanoyloxy, Benzoyloxy, das unsubstituiert ist oder substituiert durch eine oder zwei niedere Alkyl-, niedere Alkoxy-, Halogen- oder Trifluormethyl-Gruppen; oder $R_4$ und $R_5$, wenn sie vereinigt und an dasselbe Kohlenstoffatom gebunden sind, spiro-kondensiertes Cyclopentyl oder spiro-kondensiertes Cyclohexyl darstellen; n die ganze Zahl 6, 7, 8, 9 und 10 darstellt; Aryl Phenyl darstellt oder Phenyl, das mono- oder disubstituiert ist durch niederes Alkyl, niederes Alkoxy, Hydroxy, Halogen oder Trifluormethyl; Tautomere davon; oder ein pharmazeutisch annehmbares Salz davon; wobei der Ausdruck "niederes" solche mit bis zu und inklusive 7 Kohlenstoffatomen definiert.

4. Verfahren nach Anspruch 3, worin eine Verbindung der Formel II hergestellt wird, in der n die ganze Zahl 6 darstellt.

5. Verfahren nach Anspruch 3, worin eine Verbindung der Formel II hergestellt wird, worin n die ganze Zahl 7 darstellt.

6. Verfahren nach Anspruch 3, worin eine Verbindung der Formel II hergestellt wird, worin n die ganze Zahl 8 darstellt.

7. Verfahren nach Anspruch 3, worin eine Verbindung der Formel II hergestellt wird, worin n die ganze Zahl 9 darstellt.

**8.** Verfahren nach Anspruch 3, worin eine Verbindung der Formel II hergestellt wird, worin n die ganze Zahl 10 darstellt.

**9.** Verfahren nach Anspruch 3, worin eine Verbindung der Formel II hergestellt wird, worin n eine ganze Zahl wie hier definiert darstellt; $R_1$ Phenyl darstellt oder Phenyl, das mono- oder disubstituiert ist durch niederes Alkyl, niederes Alkoxy, Halogen oder Trifluormethyl; $R_4$ und $R_5$ Wasserstoff oder niederes Alkyl darstellen; ein Tautomer davon; oder ein pharmazeutisch annehmbares Salz davon, wobei der Ausdruck "niederes" solche mit bis zu und inklusive 7 Kohlenstoffatomen definiert.

**10.** Verfahren nach Anspruch 3, worin eine Verbindung der Formel II hergestellt wird, worin n eine ganze Zahl wie hier definiert darstellt; $R_1$ Phenyl darstellt oder Phenyl, das monosubstituiert ist durch niederes Alkyl, niederes Alkoxy oder Halogen; $R_4$ und $R_5$ Wasserstoff oder niederes Alkyl darstellen; ein Tautomer davon; oder ein pharmazeutisch annehmbares Salz davon, wobei der Ausdruck "niederes" solche mit bis zu und inklusive 7 Kohlenstoffatomen definiert.

**11.** Verfahren nach Anspruch 10, worin eine Verbindung der Formel II hergestellt wird, worin $R_1$ Phenyl darstellt, das in der para-Position monosubstituiert ist durch niederes Alkyl, niederes Alkoxy oder Halogen; und $R_4$ und $R_5$ Wasserstoff darstellen; ein Tautomer davon, oder ein pharmazeutisch annehmbares Salz davon, wobei der Ausdruck "niederes" solche mit bis zu und inklusive 7 Kohlenstoffatomen definiert.

**12.** Verfahren nach Anspruch 3, worin eine Verbindung der Formel II hergestellt wird, worin n eine ganze Zahl wie hier definiert darstellt, $R_1$ 2-Pyridyl darstellt, $R_4$ und $R_5$ Wasserstoff darstellen, ein Tautomer davon und ein pharmazeutisch annehmbares Salz davon.

**13.** Verfahren nach Anspruch 1, worin 2-(4-Methoxyphenyl)-2,3,6,7,8,9,10,11-octahydrocycloocta[b]-pyrazolo[3,4-d]pyridin-3(5H)-on oder ein pharmazeutisch annehmbares Salz davon hergestellt wird.

**14.** Verfahren nach Anspruch 1, worin 2-(4-Chlorphenyl)-2,3,6,7,8,9,10,11-octahydrocycloocta[b]pyrazolo-[3,4-d]pyridin-3(5H)-on oder ein pharmazeutisch annehmbares Salz davon hergestellt wird.

**15.** Verfahren nach Anspruch 1, worin 2-Pyridyl-2,3,6,7,8,9,10,11-octahydrocycloocta[b]pyrazolo[3,4-d]-pyridin-3(5H)-on oder ein pharmazeutisch annehmbares Salz davon hergestellt wird.

**16.** Verfahren zur Herstellung einer pharmazeutischen Zusammensetzung, die eine Verbindung, die nach irgendeinem der Ansprüche 1-11, 13 und 14 hergestellt wird, beigemischt zu oder in Verbindung mit einem oder mehreren pharmazeutisch geeigneten Trägern enthält.

**17.** Verfahren zur Herstellung einer pharmazeutischen Zusammensetzung, die eine Verbindung, die nach irgendeinem der Ansprüche 12 und 15 hergestellt wird, beigemischt zu oder in Verbindung mit einem oder mehreren pharmazeutisch geeigneten Trägern enthält.

**Revendications**

**Revendications pour les Etats contractants suivants : AT, BE, CH, DE, FR, GB, IT, LI, LU, NL, SE**

**1.** Composé de formule

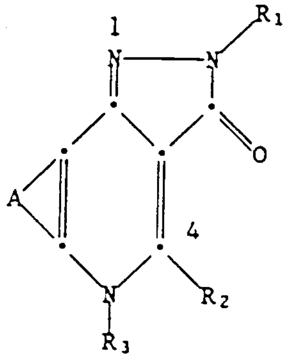

(IA)

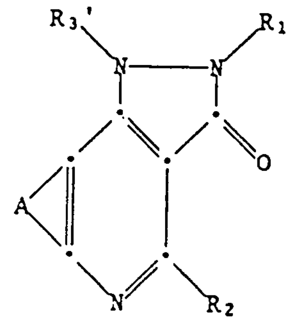

(IB)

dans laquelle A représente un groupe divalent saturé éventuellement substitué qui constitue avec les deux atomes de carbone auxquels il est fixé un cycle carboné condensé à 8, 9, 10, 11 ou 12 éléments choisis parmi cycloocténo, cyclononéno, cyclodécéno, cycloundécéno et cyclododécéno ; chacun étant non substitué ou mono-, di-, tri- ou tétrasubstitué sur des atomes de carbone de A par alkyle inférieur, alkylidène inférieur, cycloalkyle $C_3$-$C_7$, hydroxy, alcanoyloxy inférieur, benzoyloxy non substitué ou substitué par un ou deux alcoxy alkyle inférieur, alcoxy inférieur, halogène ou trifluorométhyle, oxo, alcoxy inférieur, aryle ou aryl-(alcoxy inférieur) ; et quand la disubstitution est sur le même carbone de A, ledit atome de carbone dans chaque cycle est substitué de préférence par deux groupes alkyles inférieurs ou deux groupes aryl-(alkyl inférieur), ou par un groupe alkyle inférieur ou aryl-(alkyl inférieur) et un groupe choisi parmi l'hydroxy, alcoxy inférieur, aryl-(alcoxy inférieur), le terme aryle dans les définitions ci-dessus représente le phényle ou un phényle mono- ou disubstitué par un alkyle inférieur, alcoxy inférieur, hydroxy, alcanoyloxy inférieur, benzoyloxy non substitué ou substitué par un ou deux alkyles inférieurs, alcoxy inférieur, halogène ou trifluorométhyle, et alcanoyloxy inférieur, benzoyloxy non substitué ou substitué sur un ou deux alcoxy alkyles inférieurs, alcoxy inférieur, halogène ou trifluorométhyle ou chaque cycle est disubstitué sur le même atome de carbone de A par une chaîne droite alkylène de 2 à 6 atomes de carbone formant avec l'atome de carbone auquel la chaîne alkylène est fixée un cycle spiro-condensé de 3 à 7 éléments ; ou chaque cycle est disubstitué sur des atomes de carbone adjacents par des alkylènes de 3, 4 ou 5 atomes de carbone pour former avec les deux atomes de carbone adjacents auxquels ledit groupement alkylène est fixé un cycle condensé à 5, 6 ou 7 éléments ; $R_1$ représente un alkyle inférieur, phényle, ou phényle mono- ou disubstitué par un alkyle inférieur, alcoxy inférieur, halogène ou trifluorométhyle; ou $R_1$ représente un radical hétérocyclique aromatique choisi parmi les pyridyle, quinolyle, isoquinolyle, pyrimidyle et thiazolyle, ou l'un quelconque desdits radicaux hétérocycliques mono- ou disubstitué sur le carbone par un alkyle inférieur, alcoxy inférieur ou halogène ; et $R_2$, $R_3$ et $R_3$' représentent indépendamment l'hydrogène ou un alkyle inférieur : ou un de leurs sels pharmaceutiquement acceptable, le terme "inférieur" définit les radicaux comprenant jusqu'à 7 atomes de carbone inclus.

**2.** Composé selon la revendication 1 de formules IA ou IB dans lesquelles A avec les deux atomes de carbone auxquels il est fixé représente un cycle condensé choisi parmi cycloocténo, cyclononéno, cyclodécéno, cycloundécéno et cyclododécéno dans lesquels A représente l'hexaméthylène, heptaméthylène, octaméthylène, nonaméthylène ou décaméthylène respectivement, chacun desdits cycles étant non substitué ou mono-, di-, tri- ou tétrasubstitué sur des atomes de carbone de A par alkyle inférieur, alkylidène inférieur, cycloalkyle $C_3$-$C_7$, hydroxy, alcanoyloxy inférieur, benzoyloxy non substitué ou substitué par un ou deux alcoxy alkyle inférieur, alcoxy inférieur, halogène ou trifluorométhyle, oxo, alcoxy inférieur, aryle ou aryl-(alcoxy inférieur) : et quand la disubstitution est sur le même carbone de A, ledit atome de carbone dans chaque cycle est substitué de préférence par deux groupes alkyles inférieurs ou deux groupes aryl-(alkyl inférieur), ou par un groupe alkyle inférieur ou aryl-(alkyl inférieur) et un groupe choisi parmi l'hydroxy, alcoxy inférieur, aryl-(alcoxy inférieur), le terme aryle dans les définitions ci-dessus représente le phényle ou un phényle mono- ou disubstitué par un alkyle inférieur, alcoxy inférieur, hydroxy, alcanoyloxy inférieur, benzoyloxy non substitué ou substitué par un ou deux alkyles inférieurs, alcoxy inférieur, halogène ou trifluorométhyle, ou alcanoyloxy inférieur, benzoyloxy non substitué ou substitué par un ou deux alcoxy alkyles inférieurs, alcoxy inférieur, halogène ou trifluorométhyle ou chaque cycle est disubstitué sur le même atome de carbone de A par l'éthylène, le propylène ou le pentylène pour former avec l'atome de carbone auquel la chaîne alkylène est fixée un cycle spiro-condensé cyclopropyle, cyclobutyle, cyclopentyle ou cyclohexyle : ou chaque cycle est

disubstitué sur des atomes de carbone adjacents par le propylène ou le butylène pour former avec les deux atomes de carbone adjacents auxquels ledit groupement alkylène est fixé un cycle condensé cyclopentane ou cyclohexane ; $R_1$, $R_2$, $R_3$ et $R_3$' ont les significations données dans la revendication 1 ; ou un de leurs sels pharmaceutiquement acceptables, le terme "inférieur" définit les radicaux comprenant jusqu'à 7 atomes de carbone inclus.

**3.** Composé selon la revendication 1, de formule

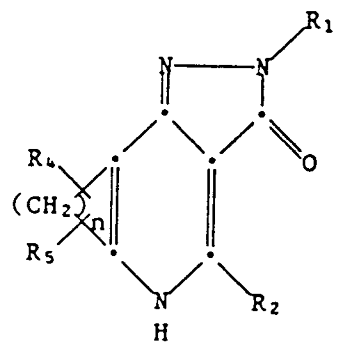

(II)

dans laquelle $R_1$ représente un alkyle inférieur, phényl ou phényl mono- ou disubstitué par un alkyle inférieur, alcoxy inférieur, halogène ou trifluorométhyle ; ou $R_1$ représente un radical hétérocyclique aromatique choisi parmi les pyridyle, quinolyle, isoquinolyle, pyrimidyle et thiazolyle, ou un quelconque desdits radicaux mono- ou di-substitués par un alkyle inférieur, alcoxy inférieur ou halogène ; $R_4$ et $R_5$ représentent indépendamment l'hydrogène, un alkyle inférieur, cycloalkyle $C_3$-$C_7$, hydroxy, alcanoyloxy inférieur, benzoyloxy non substitué ou substitué par un ou deux alcoxy alkyle inférieur, alcoxy inférieur, halogène ou trifluorométhyle, alcoxy inférieur, aryle, aryl-(alkyl inférieur) ou aryl-(alcoxy inférieur), le terme aryle dans les définitions ci-dessus représente le phényle ou un phényle mono- ou di-substitué par un alkyle inférieur, alcoxy inférieur, hydroxy, alcanoyloxy inférieur, benzoyloxy non substitué ou substitué par un ou deux alkyle inférieur, alcoxy inférieur, halogène ou trifluorométhyle ; ou $R_4$ et $R_5$ quand on les combine et fixe au même atome de carbone représente le cyclopentyle spiro-condensé ou le cyclohexyle spiro-condensé ; n représente l'entier 6, 7, 8, 9 et 10 ; aryle représente le phényle ou un phényle mono- ou di-substitué par un alkyle inférieur, alcoxy inférieur, hydroxy, halogène ou trifluorométhyle ; leurs formes tautomères ; ou un de leurs sels pharmaceutiquement acceptables, le terme "inférieur" représente des radicaux comprenant jusqu'à 7 atomes de carbone inclus.

**4.** Composé selon la revendication 3 dans lequel n représente l'entier 6.

**5.** Composé selon la revendication 3 dans lequel n représente l'entier 7.

**6.** Composé selon la revendication 3 dans lequel n représente l'entier 8.

**7.** Composé selon la revendication 3 dans lequel n représente l'entier 9.

**8.** Composé selon la revendication 3 dans lequel n représente l'entier 10.

**9.** Composé selon la revendication 3 dans lequel n représente un entier comme cela y est défini ; $R_1$ représente un phényle ou phényle monosubstitué par un alkyle inférieur, alcoxy inférieur ou halogène ; $R_4$ et $R_5$ représentent l'hydrogène ou un alkyle inférieur ; une de ses formes tautomères ; ou un de ses sels pharmaceutiquement acceptable, le terme "inférieur" définit les radicaux qui comprennent jusqu'à 7 atomes de carbone inclus.

**10.** Composé selon la revendication 3 dans lequel n représente un entier comme cela y est défini ; $R_1$ représente un phényle monosubstitué en position para par un alkyle inférieur, alcoxy inférieur ou halogène ; $R_4$ et $R_5$ représentent l'hydrogène, une de ses formes tautomères, ou un de ses sels pharmaceutiquement acceptable, le terme "inférieur" définit les radicaux qui comprennent jusqu'à 7

atomes de carbone inclus.

**11.** Composé selon la revendication 10 dans lequel $R_1$ représente un phényle mono-substitué en position para par un alkyle inférieur, alcoxy inférieur ou halogène ; et $R_4$ et $R_5$ représentent l'hydrogène, une de ses formes tautomères, ou un de ses sels pharmaceutiquement acceptable, le terme "inférieur" définit les radicaux qui comprennent jusqu'à 7 atomes de carbone inclus.

**12.** Composé selon la revendication 3 dans lequel n représente un entier comme cela y est défini, $R_1$ représente le 2-pyridyle, $R_4$ et $R_5$ représentent l'hydrogène, une de ses formes tautomères et un de ses sels pharmaceutiquement acceptables.

**13.** Composé selon la revendication 11 qui est la 2-(4-méthoxyphényl)-2,3,6,7,8,9,10,11-octahydrocycloocta[b]pyrazolo[3,4-d]pyridin-3(5H)-one ou un de ses sels pharmaceutiquement acceptables.

**14.** Composé selon la revendication 11 qui est la 2-(4-chlorophényl)-2,3,6,7,8,9,10,11-octahydrocycloocta-[b]pyrazolo[3,4-d]pyridin-3(5H)-one ou un de ses sels pharmaceutiquement acceptables.

**15.** Composé selon la revendication 12 qui est la 2-pyridyl-2,3,6,7,8,9,10,11-octahydrocycloocta[b]pyrazolo-[3,4-d]pyridin-3(5H)-one ou un de ses sels pharmaceutiquement acceptables.

**16.** Composition pharmaceutique contenant un composé selon l'une des revendications 1-11, 13 et 14, en mélange ou en combinaison avec un ou plusieurs véhicules pharmaceutiquement appropriés.

**17.** Composition pharmaceutique contenant un composé selon l'une quelconque des revendications 13 et 16, en mélange ou en combinaison avec un ou plusieurs véhicules pharmaceutiquement appropriés.

**18.** Utilisation d'un composé selon l'une quelconque des revendications 1-11, 13 et 14 pour préparer des compositions pharmaceutiques, appropriées à l'administration entérale, parentérale ou transdermique.

**19.** Utilisation d'un composé selon l'une quelconque des revendications 12 et 15 pour préparer des compositions pharmaceutiques, appropriées à l'administration entérale, parentérale ou transdermique.

**20.** Composé selon l'une quelconque des revendications 1-11, 13 et 14 pour l'utilisation comme modulateur de récepteur de benzodiazépine.

**21.** Composé selon l'une quelconque des revendications 12 et 15 pour l'utilisation comme modulateur de récepteur de benzodiazépine.

**22.** Composé selon l'une quelconque des revendications 1-11, 13 et 14 pour l'utilisation dans un procédé thérapeutique pour traiter le corps humain ou animal.

**23.** Composé selon l'une quelconque des revendications 12 et 15 pour l'utilisation dans un procédé thérapeutique du traitement du corps humain ou animal.

**24.** Procédé par analogie pour produire un composé de formule IA ou IB selon la revendication 1, dans lequel tous les symboles ont les significations données à la revendication 1, ses sels pharmaceutiquement acceptables, le terme "inférieur" définit les radicaux qui comprennent jusqu'à 7 atomes de carbone, qui consiste en :

a) mise en réaction d'un composé de formule III

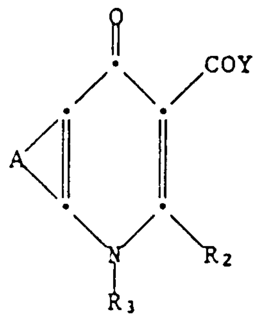

(III)

dans laquelle A, $R_2$ et $R_3$ ont les significations données antérieurement, et Y est un alcoxy inférieur ; avec un composé de formule IV

$R_3$'-NH-NH-$R_1$ (IV)

dans laquelle $R_1$ a la signification donnée antérieurement, et $R_3$' est l'hydrogène ; ou

b) mise en réaction d'un composé formule V

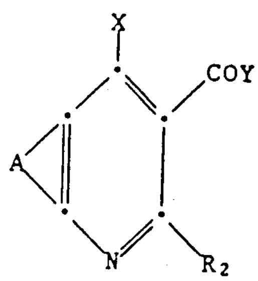

(V)

dans laquelle A et $R_2$ ont les significations données antérieurement ; X représente un hydroxy réactif éthérifié ou estérifié ; et Y représente un alcoxy inférieur ; avec un composé de formule IV dans laquelle $R_1$ a la signification donnée antérieurement, et $R_3$' représente un hydrogène ou un alkyle inférieur ; ou

c) on cyclise un composé de formule V ci-dessus, dans laquelle X est -$NR_3$'-$NHR_1$ et Y est un alcoxy inférieur ou un hydroxy ; ou X est un hydroxy, un hydroxy réactif estérifié ou éthérifié, et Y est -$NR_1NHR_3$', et dans laquelle A, $R_1$, $R_2$ et $R_3$' ont les significations données antérieurement ; ou

d) on cyclise un composé de formule V dans laquelle X est un alcoxyamino ou azido inférieur et Y est -NH-$R_1$, et A, $R_1$ et $R_2$ ont les significations définies antérieurement, ou

e) on cyclise un composé de formule VI

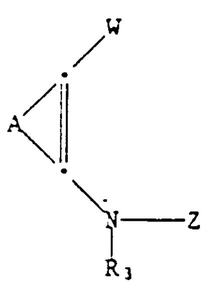

(VI)

dans laquelle W est l'hydrogène, Z est

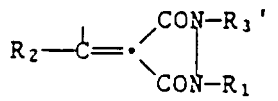

et A, $R_1$, $R_2$, $R_3$ et $R_3$' ont les significations données antérieurement ; ou

f) on cyclise un composé de formule VI ci-dessus dans laquelle W est

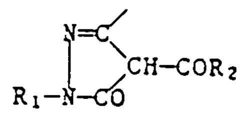

ou un de ses dérivés énamine, et Z est l'hydrogène, et A, $R_1$, $R_2$ et $R_3$ ont la même signification que celle donnée antérieurement ; ou

g) on cyclise un composé de formule VI ci-dessus dans laquelle W est

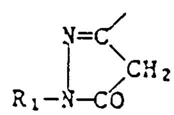

Z est $R_2CO$- ou $R_3$-N-Z est isocyano, et A, $R_1$, $R_2$ et $R_3$ ont la même signification que celle donnée antérieurement; et si on le souhaite, on transforme un composé résultant de formule Ia ou IB en un de ses sels ou on libère un composé libre à partir d'un tel sel ; ou on transforme un composé résultant en un autre composé de l'invention.

**25.** Composés préparés selon la revendication 24.

**Revendications pour les Etats contractants suivants : ES, GR**

**1.** Procédé de fabrication d'un composé de formule

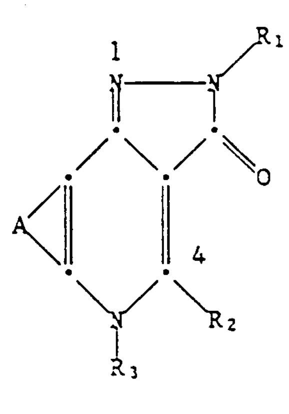

(IA)

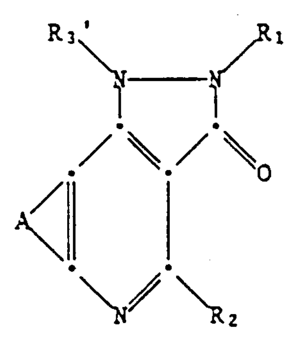

(IB)

dans laquelle A représente un groupe divalent saturé éventuellement substitué qui constitue avec les deux atomes de carbone auxquels il est fixe un cycle carboné condensé à 8, 9, 10, 11 ou 12 éléments choisis parmi cycloocténo, cyclononéno, cyclodécéno, cycloundécéno et cyclododécéno ; chacun étant non substitué ou mono-, di-, tri- ou tétrasubstitué sur des atomes de carbone de A par alkyle inférieur, alkylidène inférieur, cycloalkyle $C_3$-$C_7$, hydroxy, alcanoyloxy inférieur, benzoyloxy non substitué ou substitué par un ou deux alcoxy alkyle inférieur, alcoxy inférieur, halogène ou trifluorométhyle, oxo, alcoxy inférieur, aryle ou aryl-(alcoxy inférieur) ; et quand la disubstitution est sur le même carbone de

A, ledit atome de carbone dans chaque cycle est substitué de préférence par deux groupes alkyles inférieurs ou deux groupes aryl-(alkyl inférieur), ou par un groupe alkyle inférieur ou aryl-(alkyl inférieur) et un groupe choisi parmi l'hydroxy, alcoxy inférieur, aryl-(alcoxy inférieur), le terme aryle dans les définitions ci-dessus représente le phényle ou un phényle mono- ou disubstitué par un alkyle inférieur, alcoxy inférieur, hydroxy, alcanoyloxy inférieur, benzoyloxy non substitué ou substitué par un ou deux alkyles inférieurs, alcoxy inférieur, halogène ou trifluorométhyle, ou alcanoyloxy inférieur, benzoyloxy non substitué ou substitué par un ou deux alcoxy alkyles inférieurs, alcoxy inférieurs, halogène ou trifluorométhyle ; ou chaque cycle est disubstitué sur le même atome de carbone de A par une chaîne droite alkylène de 2 à 6 atomes de carbone formant avec l'atome de carbone auquel la chaîne alkylène est fixée un cycle spiro-condensé de 3 à 7 éléments ; ou chaque cycle est disubstitué sur des atomes de carbone adjacents par des alkylènes de 3, 4 ou 5 atomes de carbone pour former avec les deux atomes de carbone adjacents auxquels ledit groupement alkylène est fixé un cycle condensé à 5, 6 ou 7 éléments ; ou $R_1$ représente un alkyle inférieur, phényle, ou phényle mono- ou disubstitué par un alkyle inférieur, alcoxy inférieur, halogène ou trifluorométhyle: ou $R_1$ représente un radical hétérocyclique aromatique choisi parmi les pyridyle, quinolyle, isoquinolyle, pyrimidyle et thiazolyle, ou l'un quelconque desdits radicaux hétérocycliques mono- ou disubstitué sur le carbone par un alkyle inférieur, alcoxy inférieur ou halogène ; et $R_2$, $R_3$ et $R_3$' représentent indépendamment l'hydrogène ou un alkyle inférieur ; ou un de leurs sels pharmaceutiquement acceptable, le terme "inférieur" définit les radicaux comprenant jusqu'à 7 atomes de carbone inclus,

qui consiste en

a) mise en réaction d'un composé de formule III

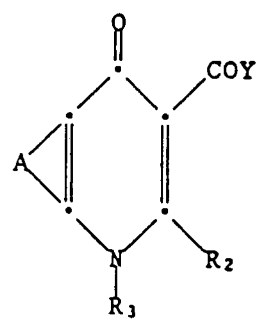

(III)

dans laquelle A, $R_2$ et $R_3$ ont les significations données antérieurement, et Y est un alcoxy inférieur ; avec un composé de formule IV

$R_3$'-NH-NH-$R_1$ (IV)

dans laquelle $R_1$ a la signification donnée antérieurement, et $R_3$' est l'hydrogène ; ou

b) mise en réaction d'un composé de formule V

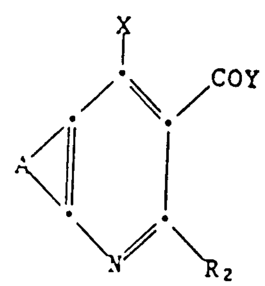

(V)

dans laquelle A et $R_2$ ont les significations données antérieurement ; X représente un hydroxy réactif éthérifié ou estérifié ; et Y représente un alcoxy inférieur ; avec un composé de formule IV dans laquelle $R_1$ a la signification donnée antérieurement, et $R_3$' représente un hydrogène ou un alkyle inférieur ; ou

c) on cyclise un composé de formule V ci-dessus, dans laquelle X est $-NR_3'-NHR_1$ et Y est un alcoxy inférieur ou un hydroxy ; ou X est un hydroxy, un hydroxy réactif estérifié ou éthérifié, et Y est $-NR_1NHR_3'$, et dans laquelle A, $R_1$, $R_2$ et $R_3'$ ont les significations données antérieurement ; ou
d) on cyclise un composé de formule V dans laquelle X est un alcoxyamino ou azido inférieur et Y est $-NH-R_1$, et A, $R_1$ et $R_2$ ont les significations définies antérieurement, ou
e) on cyclise un composé de formule VI

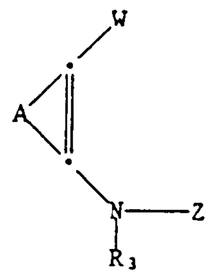

(VI)

dans laquelle W est l'hydrogène, Z est

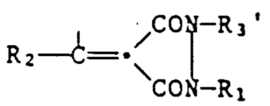

et A, $R_1$, $R_2$, $R_3$ et $R_3'$ ont les significations données antérieurement ; ou
f) on cyclise un composé de formule VI ci-dessus dans laquelle W est

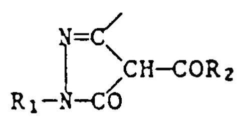

ou un de ses dérivés énamine, et Z est l'hydrogène, et A, $R_1$, $R_2$ et $R_3$ ont la même signification que celle donnée antérieurement ; ou
g) on cyclise un composé de formule VI ci-dessus dans laquelle W est

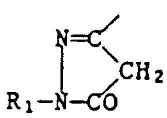

Z est $R_2CO$- ou $R_3$-N-Z est isocyano, et A, $R_1$, $R_2$ et $R_3$ ont la même signification que celle donnée antérieurement ; et si on le souhaite, on transforme un composé résultant de formule Ia ou IB en un de ses sels ou on libère un composé libre à partir d'un tel sel ; ou on transforme un composé résultant en un autre composé de l'invention.

**2.** Procédé selon la revendication 1, dans lequel on prépare un composé de formule IA ou IB dans lesquelles A avec les deux atomes de carbone auxquels il est fixé représente un cycle condensé choisi parmi cycloocténo, cyclononéno, cyclodécéno, cycloundécéno et cyclododécéno dans lesquels A représente l'hexaméthylène, heptaméthylène, octaméthylène, nonaméthylène ou décaméthylène respectivement ; chacun desdits cycles étant non substitué ou mono-, di-, tri- ou tétra-substitué sur des atomes de carbone de A par alkyle inférieur, alkylidène inférieur, cycloalkyle $C_3$-$C_7$, hydroxy, alcanoyloxy inférieur, benzoyloxy non substitué ou substitué par un ou deux alcoxy alkyle inférieur, alcoxy inférieur, halogène ou trifluorométhyle, oxo, alcoxy inférieur, aryle ou aryl-(alcoxy inférieur) ; et quand la

disubstitution est sur le même carbone de A, ledit atome de carbone dans chaque cycle est substitué de préférence par deux groupes alkyles inférieurs ou deux groupes aryl-(alkyl inférieur), ou par un groupe alkyle inférieur ou aryl-(alkyl inférieur) et un groupe choisi parmi l'hydroxy, alcoxy inférieur, aryl-(alcoxy inférieur), le terme aryle dans les définitions ci-dessus représente le phényle ou un phényle mono- ou disubstitué par un alkyle inférieur, alcoxy inférieur, hydroxy, alcanoyloxy inférieur, benzoyloxy non substitué ou substitué par un ou deux alkyles inférieurs, alcoxy inférieur, halogène ou trifluorométhyle, ou alcanoyloxy inférieur, benzoyloxy non substitué ou substitué par un ou deux alcoxy alkyles inférieurs, alcoxy inférieur, halogène ou trifluorométhyle ou chaque cycle est disubstitué sur le même atome de carbone de A par l'éthylène, le propylène ou le pentylène pour former avec l'atome de carbone auquel la chaîne alkylène est fixée un cycle spiro-condensé cyclopropyle, cyclobutyle, cyclopentyle ou cyclohexyle ; ou chaque cycle est disubstitué sur des atomes de carbone adjacents par le propylène ou le butylène pour former avec les deux atomes de carbone adjacents auxquels ledit groupement alkylène est fixé un cycle condensé cyclopentane ou cyclohexane ; $R_1$, $R_2$, $R_3$ et $R_3$' ont les significations données dans la revendication 1 ; ou un de leurs sels pharmaceutiquement acceptables, le terme "inférieur" définit les radicaux comprenant jusqu'à 7 atomes de carbone inclus.

**3.** Procédé selon la revendication 1 dans lequel on prépare un composé de formule II

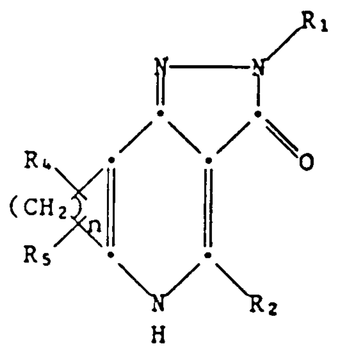

(II)

dans laquelle $R_1$ représente un alkyle inférieur, phényl ou phényl mono- ou disubstitué par un alkyle inférieur, alcoxy inférieur, halogène ou trifluorométhyle ; ou $R_1$ représente un radical hétérocyclique aromatique choisi parmi les pyridyle, quinolyle, isoquinolyle, pyrimidyle et thiazolyle, ou un quelconque desdits radicaux mono- ou di-substitués par un alkyle inférieur, alcoxy inférieur ou halogène ; $R_4$ et $R_5$ représentent indépendamment l'hydrogène, un alkyle inférieur, cycloalkyle $C_3$-$C_7$, hydroxy, alcanoyloxy inférieur, benzoyloxy non substitué ou substitué par un ou deux alcoxyalkyle inférieur, alcoxy inférieur, halogène ou trifluorométhyle, alcoxy inférieur, aryle, aryl-(alkyl inférieur) ou aryl-(alcoxy inférieur), le terme aryle dans les définitions ci-dessus représente le phényle ou un phényle mono- ou di-substitué par un alkyle inférieur, alcoxy inférieur, hydroxy, alcanoyloxy inférieur, benzoyloxy non substitué ou substitué par un ou deux alkyle inférieur, alcoxy inférieur, halogène ou trifluorométhyle ; ou $R_4$ et $R_5$ quand on les combine ou fixe au même atome de carbone représente le cyclopentyle spiro-condensé ou le cyclohexyle spiro-condensé ; n représente l'entier 6, 7, 8, 9 et 10 ; aryle représente le phényle ou un phényle mono- ou di-substitué par un alkyle inférieur, alcoxy inférieur, hydroxy, halogène ou trifluorométhyle ; leurs formes tautomères ; ou un de leurs sels pharmaceutiquement acceptables, le terme "inférieur" représente des radicaux comprenant jusqu'à 7 atomes de carbone inclus.

**4.** Procédé selon la revendication 3, dans lequel on prépare un composé de formule II dans laquelle n représente l'entier 6.

**5.** Procédé selon la revendication 3, dans lequel on prépare un composé de formule II dans laquelle n représente l'entier 7.

**6.** Procédé selon la revendication 3, dans lequel on prépare un composé de formule II dans laquelle n représente l'entier 8.

**7.** Procédé selon la revendication 3, dans lequel on prépare un composé de formule II dans laquelle n représente l'entier 9.

**8.** Procédé selon la revendication 3, dans lequel on prépare un composé de formule II dans laquelle n représente l'entier 10.

**9.** Procédé selon la revendication 3 dans lequel on prépare un composé de formule II dans laquelle n représente un entier comme cela y est défini ; $R_1$ représente un phényle ou phényle monosubstitué par un alkyle inférieur, alcoxy inférieur, halogène ou trifluorométhyle: $R_4$ et $R_5$ représentent l'hydrogène ou un alkyle inférieur ; une de ses formes tautomères ; ou un de ses sels pharmaceutiquement acceptables, le terme "inférieur" définit les radicaux qui comprennent jusqu'à 7 atomes de carbone inclus.

**10.** Procédé selon la revendication 3, dans lequel on prépare un composé de formule II dans laquelle n représente un entier comme cela y est défini ; $R_1$ représente un phényle monosubstitué en position para par un alkyle inférieur, alcoxy inférieur ou halogène ; $R_4$ et $R_5$ représentent l'hydrogène, une de ses formes tautomères ; ou un de ses sels pharmaceutiquement acceptables, le terme "inférieur" définit les radicaux qui comprennent jusqu'à 7 atomes de carbone inclus.

**11.** Procédé selon la revendication 10, dans lequel on prépare un composé de formule II dans laquelle $R_1$ représente un phényle mono-substitué en position para par un alkyle inférieur, alcoxy inférieur ou halogène ; et $R_4$ et $R_5$ représentent l'hydrogène, un de ses formes tautomères, ou un de ses sels pharmaceutiquement acceptables, le terme "inférieur" définit les radicaux qui comprennent jusqu'à 7 atomes de carbone inclus.

**12.** Procédé selon la revendication 3, dans lequel on prépare un composé de formule II dans laquelle n représente un entier comme cela y est défini, $R_1$ représente le 2-pyridyle, $R_4$ et $R_5$ représentent l'hydrogène, une de ses formes tautomères et un de ses sels pharmaceutiquement acceptables.

**13.** Procédé selon la revendication 1 dans lequel on prépare la 2-(4-méthoxyphényl)-2,3,6,7,8,9,10,11-octahydrocycloocta[b]pyrazolo[3,4-d]pyridin-3(5H)-one ou un de ses sels pharmaceutiquement acceptables.

**14.** Procédé selon la revendication 1 dans lequel on prépare la 2-(4-chlorophényl)-2,3,6,7,8,9,10,11-octahydrocycloocta[b]pyrazolo[3,4-d]-pyridin-3(5H)-one ou un de ses sels pharmaceutiquement acceptables.

**15.** Procédé selon la revendication 1, dans lequel on prépare la 2-pyridyl-2,3,6,7,8,9,10,11-octahydrocycloocta[b]pyrazolo[3,4-d]pyridin-3(5H)-one ou un de ses sels pharmaceutiquement acceptables.

**16.** Procédé de production d'une composition pharmaceutique contenant un composé préparé selon l'une quelconque des revendications 1-11, 13 et 14, en mélange ou en combinaison avec un ou plusieurs véhicules pharmaceutiquement appropriés.

**17.** Procédé de production d'une composition pharmaceutique contenant un composé préparé selon l'une quelconque des revendications 12 et 15, en mélange ou en combinaison avec un ou plusieurs véhicules pharmaceutiquement appropriés.